# EUROPÄISCHE PATENTSCHRIFT

(11) **EP 2 935 595 B1**
(45) Veröffentlichungstag und Bekanntmachung des Hinweises auf die Patenterteilung: **08.04.2020**
(21) Anmeldenummer: 13807989.2
(22) Anmeldetag: 16.12.2013
(51) Int. Cl.: C12P 5/02, C12M 1/00, C12M 1/107

(54) **VERFAHREN UND ANLAGE ZUR HERSTELLUNG VON BIOGAS AUS LIGNOCELLULOSEHALTIGER BIOMASSE**
METHOD AND PLANT FOR PRODUCING BIOGAS FROM LIGNOCELLULOSE-CONTAINING BIOMASS
PROCEDE ET SYSTEME POUR LA PRODUCTION DE BIOGAZ À PARTIR DE BIOMASSE LIGNOCELLULOSIQUE

(30) Priorität: 21.12.2012 DE 102012112898
(43) Veröffentlichungstag der Anmeldung: 28.10.2015
(73) Patentinhaber: Verbio Vereinigte Bioenergie AG, 04109 Leipzig (DE)
(72) Erfinder: LÜDTKE, Oliver, 04416 Markkleeberg (DE); SCHLIMBACH, Michael, 06120 Halle (DE); FICHTER,Enrico, 04315 Leipzig (DE); HORN, Jens, 04277 Leipzig (DE); POLLERT, Georg, 13465 Berlin (DE); KÜHLING, Jan, 06114 Halle (DE)
(74) Vertreter: Letzelter, Felix Phillip
(86) Internationale Anmeldenummer: PCT/EP2013/076630
(87) Internationale Veröffentlichungsnummer: WO 2014/095669

(56) Entgegenhaltungen:
- EP-A1- 1 978 086
- WO-A1-01/60752
- WO-A1-2006/042551
- WO-A1-2009/000305
- DE-A1-102008 024 388
- BAUER A ET AL: "Analysis of methane potentials of steam-exploded wheat straw and estimation of energy yields of combined ethanol and methane production", JOURNAL OF BIOTECHNOLOGY, ELSEVIER SCIENCE PUBLISHERS, AMSTERDAM, NL, Bd. 142, Nr. 1, 1. Juni 2009 (2009-06-01), Seiten 50-55, XP026139436, ISSN: 0168-1656, DOI: 10.1016/J.JBIOTEC.2009.01.017 [gefunden am 2009-02-06]
- Ventury GmbH, Präsentation "Thermische Druckwechselkonditionierung" , gehalten am 05.12.2012 im Rahmen des vom Bundesministerium für Bildung und Forschung geförderten Projekts "Chemnitz FutureGas"

## Beschreibung

### HINTERGRUND DER ERFINDUNG

### Technisches Gebiet

Die Erfindung bezieht sich auf ein ganzheitlich zusammenwirkendes Verfahren zur energie- und ressourcenschonenden Herstellung von Biogas aus lignocellulosehaltiger Biomasse.

Wissenschaftlich und gesellschaftlich anerkannt ist die Notwendigkeit einer Verringerung menschlich verursachter Emissionen von Treibhausgasen wie insbesondere von Kohlendioxid (CO2), um dem aktuell stattfindenden Klimawandel entgegenzuwirken. Gleichzeitig steigt durch die wachsende Weltbevölkerung der Bedarf an Nahrung, Trinkwasser sowie Energie bzw. Endenergieträgern und Kraftstoffen. Neuartige Verfahren und Produktionsprozesse müssen an diesen aktuellen Anforderungen ausgerichtet werden. Die vorliegende Erfindung wird diesem Anspruch gerecht und bietet ein Verfahren zur Herstellung von Biogas aus bisher wenig genutzter lignocellulosehaltiger, nicht nahrungsmittelfähiger Biomasse. Das Verfahren kann Biogas durch Minimierung des Energie- und Wasserbedarfs ressourcenschonend und mit sehr hohem Treibhausgasminderungspotenzial im Vergleich zu fossilen Energieträgern zur Verfügung stellen.

### Beschreibung des Standes der Technik

Die Herstellung von Biogas aus lignocellulosehaltiger Biomasse erfolgt grundsätzlich wie auch die Vergärung von Gülle und nachwachsenden Rohstoffen (Nawaros) mit Hilfe der Schritte: Aufbereitung der lignocellulosehaltigen Biomasse, Anmischung mit Wasser, anaerobe Vergärung durch Mikroorganismen über die Zwischenschritte Hydrolyse, Acidogenese, Acetogenese und Methanogenese sowie in der Regel Aufbereitung der Gärreste und des erzeugten Biogases.

Dabei ist es für eine hohe Biogasausbeute nützlich lignocellulosehaltige Biomasse wie zum Beispiel Stroh so zu behandeln, dass das in den Zellwänden eingelagerte Lignin aufgebrochen und/oder abgebaut wird und dadurch die enthaltene Cellulose und Hemicellulose für den mikrobiellen anaeroben Abbau besser zugänglich sind. Hierzu bestehen eine Reihe von allgemein bekannten Techniken, die beispielsweise auf den Prinzipien der mechanischen Zerkleinerung, thermischen, chemischen oder enzymatischen Behandlung sowie biologischen Aufspaltung mit Pilzen beruhen.

Darüber hinaus ist auch bei der Herstellung von Biogas aus lignocellulosehaltiger Biomasse eine prozessinterne Wasserrezirkulation bekannt. Entsprechende Verfahren zur Rückgewinnung von Prozesswasser beruhen zum Beispiel auf einer einfachen Phasentrennung mittels Pressen oder Dekanterzentrifugen.

Ein in der Biogastechnik bekanntes Problem bei der Vergärung faserhaltiger Rohstoffe, insbesondere Stroh, ist die Bildung von Schwimmschichten während des Gärprozesses. Dem wird durch starke Zerkleinerung des faserhaltigen Materials bzw. durch mechanische Zerstörung der Schwimmschicht begegnet.

Ferner ist bekannt, dass bei der Vergärung von überwiegend lignocellulosehaltigem Material nicht alle für eine weitreichende mikrobiologische Umsetzung notwendigen Nährstoffe vorhanden sind und dem Gärprozess zugeführt werden müssen. Unklar ist jedoch in der Regel, in welcher Konzentration die Nährstoffe vorhanden sein müssen. Empfehlungen aus der Literatur beziehen sich auf die Vergärung bisher bekannter Substrate wie Nawaros und erstrecken sich über einen sehr weiten Konzentrationsbereich von mehreren Zehnerpotenzen.

In WO 2009/016082 A2 ist zum Beispiel ein Verfahren zur Konversion von Biomasse aus nachwachsenden Rohstoffen beschrieben, bei welchem der Gärrest aus einem ersten anaeroben Fermenter einer Fest-Flüssig-Phasentrennung, bevorzugt durch Pressen, Zentrifugation oder Siebung, unterzogen wird und anschließend die abgetrennte Feststoffphase durch eine Thermodruckhydrolyse bei mindestens 170°C und einem Druck von mindestens 1MPa und einer bevorzugten Dauer zwischen 10min und 120min behandelt wird, um sie erneut einem anaeroben Fermenter zuzuführen. Dabei sollen durch den ersten Gärvorgang mit einer durchschnittlichen Dauer von 25 Tagen, ca. 40% der organischen Ausgangsmasse abgebaut und in Biogas umgewandelt werden. In dem zweiten Gärvorgang, nach der Thermodruckhydrolyse, mit einer Dauer von beispielsweise 20 Tagen, soll sich der Gesamtabbaugrad auf 80% steigern lassen, wobei als Rohstoff eine Silage aus nicht agrarischen Reststoffen von 60% Mais und 40% Roggen zusammen mit Gülle eingesetzt wurde.

Das in WO 2009/016082 A2 beschriebene Verfahren ist explizit auf nachwachsende Rohstoffe bezogen, zu welcher im Allgemeinen speziell angebaute Biomassen wie zum Beispiel Maisganzpflanzen gerechnet werden. Es hat insbesondere bei der Anwendung auf stark lignocellulosehaltige Biomasse wie zum Beispiel Stroh eine Reihe von Nachteilen. So wird das Problem der Schwimmschichtbildung bei der Fermentation nicht berücksichtigt. Ferner ist das beschriebene Aufschlussverfahren sehr energie- und kostenintensiv. Zunächst erfordert die Behandlung von großen Gärrestmengen mit Schneckenpressen zur starken Anreicherung der Trockensubstanz (TS) einen hohen Energiebedarf sowie Wartungs- und Investitionsaufwand. Nach der Thermodruckhydrolyse muss den im TS-Gehalt angereicherten Gärresten die zuvor abgetrennte Flüssigphase zumindest anteilig wieder hinzugefügt werden, um einen für eine Fermentation günstigen TS-Gehalt einzustellen. Somit wird die TS-Anreicherung ausschließlich für die ohnehin energieintensive Thermodruckhydrolyse vorgesehen. Bei dieser muss eine etwa 30%ige Feststoffphase gefördert, aufgeheizt und für eine anschließende Fermentation abgekühlt werden. Eine solche, vorwiegend aus Fasern bestehende Feststoffphase ist zumindest bei der Verwendung von Stroh bzw. Strohgärrest mit herkömmlichen Pumpen nicht mehr pumpfähig. Darüber hinaus ist eine Wärmeübertragung der sehr großen Energiemengen in eine solche Feststoffphase indirekt über einen vorgeschlagenen Abgaswärmetauscher aus dem Abgas eines BHKW mit einem sehr großen apparativen Aufwand und entsprechend hohen Kosten verbunden. Zudem setzt dieses Konzept zwingend eine direkte Verbrennung des erzeugten Biogases in der Anlage voraus. Eine alternative Erwärmung durch hochwertigen Direktdampf von mindestens 1,0MPa würde für den beschriebenen Chargenbetrieb bei der Entspannung deutlich abgewertet werden und, da keine weitere Nutzung aufgezeigt wird, verloren gehen. Das Verfahren beschreibt zudem den gemischten Einsatz von Biomasse und weiteren für die Methanogenese notwendigen Ausgangsstoffen wie z. B. Gülle und/oder Klärschlämmen und ist für eine anaerobe Fermentation, ausschließlich von lignocellulosehaltiger Biomasse wie z. B. Stroh nicht geeignet. Dies wird auch daran ersichtlich, dass die für eine effiziente Strohvergärung notwendige prozessinterne Rückführung einer flüssigen Phase nicht beschrieben wird.

Die Patentschrift WO 2009/000305 A1 beschreibt eine Anlage und ein Verfahren zur Erzeugung von Biogas in einem Fermenter mittels anaerober bakterieller Vergärung von Biomasse, insbesondere Stroh, wobei ein chemischer, mechanischer und/oder thermischer Aufschluss durchgeführt wird und die Anlage bevorzugt im Feststoffvergärungsverfahren arbeitet. Zum Aufschluss von ligninhaltigen nachwachsenden Rohstoffen wird eine Einrichtung zur Sattdampfbehandlung von Strohballen oder losem lignocellulosehaltigem Material detailliert beschrieben. Diese Sattdampfbehandlung arbeitet chargenweise mit sehr energiereichem Dampf von 20 bis 30bar. Bestandteil dieser Sattdampfbehandlung ist die schlagartige Entspannung, wodurch die Lignocellulosefasern zerrissen werden sollen. Dieses Prinzip wird in der Fachwelt allgemein als "steam explosion" bezeichnet. Für die anschließende Vergärung wird vorzugsweise auf die Feststofffermentation verwiesen ohne jedoch eine Nassfermentation auszuschließen. Demgegenüber wird an anderer Stelle explizit auf die Probleme der Schwimmschichtbildung bei einer Nassfermentation von Stroh verwiesen. Demnach komme es für die Nassvergärung nicht in Betracht, weil dieses aufschwimmt, in der Regel Ab- bzw. Überläufe verstopft oder sich um die Paddel bzw. Propeller in Nassfermentationsanlagen wickeln würde.

Das beschriebene Verfahren aus WO 2009/000305 A1 ist unter energetischer und wirtschaftlicher Betrachtung weniger gut geeignet, insbesondere weil die beschriebenen chargenweise betriebenen thermischen Aufschlussverfahren nicht über Mittel der Energierückgewinnung aus dem Abdampf der Entspannung verfügen. Durch das beschriebene Verfahren bzw. die Anlage muss das lignocellulosehaltige Substrat innerhalb der gesamten Anlage als Feststoff transportiert werden. Dies ist logistisch und insbesondere apparatetechnisch wesentlich aufwändiger und damit kostenintensiver als der Transport einer pumpfähigen Suspension, so wie sie zum Beispiel bei Nassfermentationen vorliegt. Darüber hinaus sind keine Mittel vorhanden, um eine Biogaserzeugung aus Stroh in einer Nassfermentationsanlage durchzuführen, es wird sogar das technische Problem beschrieben, dass Stroh Schwimmschichten bildet und deshalb nicht für die Nassfermentation geeignet sei. Ein weiterer Nachteil erwächst aus dem beschriebenen Chargenbetrieb der quasikontinuierlichen Trockenfermentation in Garagenfermentern. Einerseits entstehen beim Entleeren der Fermenter Methanverluste. Zudem müssen die dabei auftretenden relevanten Methan- und Faulgasemissionen aus Klimaschutz- und emissionsrechtlichen Gründen verhindert werden, was zwangsläufig eine Einhausung der gesamten Anlage und ein Abluftreinigungssystem zur Folge hat und die Wirtschaftlichkeit des gesamten Verfahrens mindert.

Außerhalb des technischen Gebiets der Biogasherstellung wird in der EP 0 377 832 A2 ein Verfahren zur Behandlung von Biomasse zur biologischen Abwasserreinigung beschrieben. Mit dem Ziel einer nahezu vollständigen Hydrolyse wird die flüssige Biomasse in einer Thermodruckhydrolyse, einstufig oder mehrstufig, zwischen 150°C und 300°C gegebenenfalls unter Dosierung von Zugabestoffen wie anorganischen Katalysatoren für 30min bis 120min behandelt. Dabei wird in einem Wärmetauscher der zu hydrolysierenden Biomasse Wärme vom Hydrolysat übertragen. Anschließend erfolgt die Trennung in eine Feststoffphase als Reststoffmasse sowie in eine Monomere enthaltende Flüssigphase als organischer Wertstoff.

Das Verfahren in der genannten Patentliteratur EP 0 377 832 A2 behandelt weder die Biogasherstellung noch eine Fermentation, sondern das einer Abwasserreinigung mit Hilfe einer Thermodruckhydrolyse. Die angegebenen Prozessparameter sind für eine anschließende Vergärung nicht sinnvoll. Bei den beschriebenen Bedingungen entstehen speziell aus hemicellulosehaltiger Biomasse wie zum Beispiel Stroh nicht nur Monomere, sondern insbesondere auch schwer abbaubare aromatische Verbindungen wie zum Beispiel HMF und Furfurale. Diese können zu einer Inhibierung der biologischen Abbauprozesse führen. Für eine anschließende Vergärung, insbesondere wenn ein großer Teil der Prozessflüssigkeit rezirkuliert werden soll, ist dieses Verfahren demnach ungeeignet.

Ebenfalls aus einem anderen technischen Gebiet ist der Einsatz einer biologischen Behandlung von lignocellulosehaltigem Substrat mit Weißfäulepilzen bekannt. DE 25 43 939 A1 beschreibt ein Verfahren zur Futterwertsteigerung von Stroh. Dabei wird das mit Dampf pasteurisierte Stroh mit Weißfäulepilzkulturen versetzt. Diese sollen durch überwiegenden Ligninabbau die Cellulose freilegen und damit die Verdaulichkeit des Strohs erhöhen. In Versuchen wurde jedoch festgestellt, dass mit 20%-35% auch relevante Mengen der Cellulose abgebaut wurden.

Im Hinblick auf eine Biogasproduktion aus Stroh ist dieses Verfahren nicht direkt übertragbar, da die hohen Celluloseverluste unweigerlich die Ökonomie des gesamten Verfahrens verringern würden.

Der Einsatz einer biologischen Behandlung von lignocellulosehaltigem Substrat mit Weißfäulepilzen im Zusammenhang mit einer anaeroben Biogasfermentation ist in DE 44 14 459 A1 beschrieben. Das Verfahren fokussiert primär auf die mehrstufige Biogaserzeugung aus Abfällen mit enthaltenden organischen Stoffen, wie Hausmüll oder dergleichen, und der damit einhergehenden Mineralisierung und Hygienisierung. Als eine mögliche Verfahrensvariante wird der Ligninabbau in einer der Hydrolyse vorgeschalteten aeroben Stufe beschrieben, wodurch der nachfolgende anaerobe Celluloseabbau verbessert werden soll. Dieser Ligninabbau soll durch die Einstellung eines pH-Werts von 4,2 bis 4,6 und durch den Einsatz von zugeführten Weißfäulepilzen ohne signifikanten Abbau von Cellulose oder anderem, leichter abbaubarem Substrat erfolgen.

Das in DE 44 14 459 A1 beschriebene Verfahren ist nicht direkt auf eine Verwertung von überwiegend lignocellulosehaltiger Biomasse übertragbar. Zur Ausbildung von Schwimmschichten, wie sie speziell bei der Verarbeitung von Stroh zu erwarten ist, wird keine praktikable Lösung aufgezeigt. Es wird vielmehr davon ausgegangen, dass durch die Einstellung eines TS-Gehalts von vorzugsweise 15% bis 20% im Fermenter keine Segregation erfolgt, was bei einer Strohsuspension nachweislich nicht der Fall ist. Ein weiterer Nachteil besteht in der für eine Vergleichmäßigung des Prozesses beabsichtigten Zuführung von leicht abbaubarem organischem Material wie zum Beispiel Melasse und/oder Klärschlamm, wodurch sich das beschriebene Verfahren erheblich von einer anspruchsvolleren lignocellulosebasierten Vergärung entfernt.

Die Neigung zur Bildung von Schwimmschichten bei der Vergärung von lignocellulosehaltigem Material ist bekannt. Diese Neigung wird der die Fasern umschließenden Wachsschicht zugeschrieben, welche das Durchfeuchten der Lignocellulosefasern verhindert. Zur Beseitigung von Schwimmschichten bei der anaeroben Vergärung sind mechanische Verfahren bekannt, welche die Schwimmschicht mit Rühr- oder Strahlrührwerkzeugen zerstören bzw. auflösen oder durch die induzierte Strömung eine Schwimmschichtbildung verhindern. Eine solche Vorrichtung wird in DE 7901636 U1 für Güllesilos in landwirtschaftlichen Betrieben beschrieben. Bei dieser Vorrichtung werden Schwimmschichten, die anteilig aus aufschwimmendem Stroh gebildet sein können, zusammen mit Gülle durch einen Überlauf in einer Nebenkammer gesammelt und bei der anschließenden Zwangsförderung zurück in den Fermenter durch eine mit Reißwerkzeugen ausgerüstete Pumpe vollständig zerstört.

Nachteilig bei dieser Lösung wie auch beim Einsatz von speziellen Rührern ist die Bekämpfung der Schwimmschichten unter Einsatz von wartungsbehafteter Förder- oder Rührtechnik und damit verbundenem zusätzlichen, oftmals signifikanten Energiebedarf. Dabei wird das Problem nicht aber die eigentliche Ursache der Bildung der Schwimmschichten bekämpft.

Die WO 2006/042551 betrifft ein Verfahren und eine Vorrichtung zur Umwandlung von organischen Abfallstoffen in Biogas. Bei den zu verarbeitenden organischen Abfallstoffen handelt es sich insbesondere um Dung oder Tierkörperrückstände, aber auch pflanzliche Rückstände werden als Biomaterial in Betracht gezogen. Bei dem beschriebenen Verfahren wird zunächst eine Fermentation des organischen Abfallstoffes in einem ersten Reaktor vorgesehen, gefolgt von einer Hydrolyse des so fermentierten organischen Abfallstoffes in einem anaeroben Hydrolysetank, gefolgt von einer weiteren Fermentation in einem zweiten Reaktor.

Die WO 01/60752 A1 beschreibt ein kontinuierliches Verfahren, das eine Nassoxidation oder eine Dampfexplosion umfasst, um lignocellulosehaltige Biomasse fermentativ in Ethanol umzuwandeln. Dabei soll das gesamte oder ein Teil des Prozesswassers recycelt werden. Das Abwasser aus dem Ethanol-Fermentationsschritt kann einem anaeroben Fermentationsschritt unterworfen werden, bei dem Methan erzeugt wird.

Die EP 1 978 086 A1 offenbart ein Verfahren zur Herstellung von Biogas oder Bioethanol aus lignocellulosehaltiger Biomasse. Das Verfahren umfasst das Behandeln der Biomasse durch einen Schredder, das Erhitzen und thermische Hydrolysieren der zerkleinerten Biomasse in einer Hochdruckmikrowelle bei 150-250 °C. Der Fermentationsprozess wird mikrobiologisch und/oder enzymatisch in einem Hochleistungsreaktor mit einer Fermenterbeladung von weniger als 5 kg organischer Trockensubstanz/m³ bei 40 °C durchgeführt.

### AUFGABE UND LÖSUNG

Bisher bekannten Verfahren zur Biogasproduktion aus lignocellulosehaltiger Biomasse ist gemein, dass sie gegebenenfalls für eine Verarbeitung von faserhaltigen Stoffen wie Maisganzpflanzen möglich, jedoch für eine Verarbeitung von stärker lignifiziertem Material wie zum Beispiel Stroh als Hauptsubstrat bzw. bei einer Strohmonovergärung ungeeignet oder durch sehr hohe Anlagen- und/oder Betriebskosten wenig wirtschaftlich sind.

Existierende Verfahren aus anderen technischen Bereichen zur Verwertung von lignocellulosehaltiger Biomasse werden den speziellen Anforderungen einer Biogasproduktion nicht gerecht und sind nicht direkt übertragbar bzw. in ihrer ursprünglichen Verfahrensform zu kostenintensiv.

Der vorliegenden Erfindung lag somit die Aufgabe zugrunde, ein besonders energie- und ressourcenschonendes und damit kosteneffizientes und mit geringen CO2-Emissionen behaftetes Verfahren zur Herstellung von Biogas aus lignocellulosehaltiger Biomasse sowie eine Anlage zur Durchführung dieses Verfahrens bereitzustellen. Zur Lösung dieser Aufgabe stellt die Erfindung ein Verfahren gemäß Anspruch 1 bereit, wonach das Verfahren zur Herstellung von Biogas aus lignocellulosehaltiger Biomasse, vorzugsweise aus Stroh, folgende Schritte umfasst:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, für eine Zeitdauer von 5-30min insbesondere mit Prozesswasser und/oder unter Zuhilfenahme von Dampf;
   a1) anaerobe Fermentation der angemischten lignocellulosehaltigen Biomasse mithilfe von Mikroorganismen und Gewinnung des entstehenden Biogases
   a2) Durchführung eines im Gegenstromprinzip arbeitenden Wärmeaustauschs zwischen der in Schritt a1) erhaltenen einer ersten anaeroben Fermentation unterzogenen lignocellulosehaltigen Biomasse und der in Schritt b) erhaltenen thermisch behandelten lignocellulosehaltigen Biomasse;
b) weitere Erhitzung der aus a2) im Gegenstrom erwärmten lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss;
c) anaerobe Fermentation der thermisch behandelten lignocellulosehaltigen Biomasse mithilfe von Mikroorganismen und Gewinnung des entstehenden Biogases;
d) wobei der Ablauf nach der Fermentation c), zumindest anteilig einer Phasentrennung unterzogen wird und
e) eine flüssige Phase aus d) in Form von Prozesswasser gewonnen wird und diese zum Anmischen von lignocellulosehaltiger Biomasse rückgeführt wird derart, dass die Zufuhr an Frischwasser im Biogaserzeugungsprozess und/oder zum Anmischen zumindest wesentlich reduziert wird oder vollständig entfällt sowie
f) eine feststoffreiche Phase aus d) zum Beispiel als organischer Dünger anfällt. Vorteilhafte Weiterbildungen sind in den abhängigen Ansprüchen genannt.

Der Wortlaut aller Ansprüche wird durch die Bezugnahme zum Inhalt der Beschreibung gemacht.

### Beschreibung der Lösung

Die energie- und ressourcenschonende Herstellung von Biogas durch anaerobe Vergärung lignocellulosehaltiger Biomasse ist nur dann möglich, wenn die Biogasausbeute hinreichend hoch ist. Einerseits weil nur dann der Energieertrag in Form von Biogas entsprechend hoch ist. Zudem aber insbesondere auch weil gleichzeitig soviel Trockensubstanz abgebaut wird, dass der Prozesswasserbedarf für das Anmischen des Strohs aus der Fest-Flüssig-Trennung des Gärrests fast vollständig oder sogar komplett gedeckt werden kann. Dies ist bei einer entsprechenden Vorbehandlung der lignocellulosehaltigen Biomasse, insbesondere in Form von Stroh, in mehreren Verfahrensschritten mit anschließender Nassfermentation einer bereiteten Strohsuspension, gegebenenfalls in mehreren nachfolgenden Stufen, einer ausreichend mir Nährstoffen versorgten anaeroben Fermentation, in Kombination mit einem energetisch effizienten, thermischen Aufschluss und gegebenenfalls mit einem aeroben biologischen Ligninabbau möglich.

Im Einzelnen konnten mit dem erfindungsgemäßen, ganzheitlich zusammenwirkenden Lösungskonzept die folgenden Vorteile erzielt werden:
Durch eine Zerkleinerung der lignocellulosehaltigen Biomasse wie zum Beispiel Stroh, Gräser, Holz oder anderen agrarischen und forstwirtschaftlichen Reststoffen im Bereich von 1mm bis 10mm Partikelgröße ist das Verhältnis zwischen dem Energieaufwand für die Zerkleinerung und dem Ertrag an Biogas optimal.

Durch eine kurzzeitige Temperierung der zerkleinerten lignocellulosehaltigen Biomasse wie zum Beispiel Stroh auf ca. 80°C in feuchter Umgebung wird eine Bildung von Schwimmschichten im anaeroben Fermentationsprozess stark reduziert oder sogar vollständig vermieden.

Durch eine physikalisch-thermische Behandlung von lignocellulosehaltiger Biomasse bei einer Temperatur von 130-200°C und anschließender Vergärung wird die Biogasausbeute signifikant gesteigert und die Verfügbarkeit von Stickstoff verbessert.

Durch die Wahl der Temperatur bis 200°C ist das Verhältnis zwischen dem Energieaufwand für die physikalisch-thermische Behandlung und dem erzielbaren Mehrertrag an Biogas besonders wirtschaftlich.

Durch die Einstellung des Gehalts von Makro- und Mikronährstoffen im Fermentationssystem stellt die Erfindung als ein wesentliches Kernelement die Mittel für eine Monovergärung von lignocellulosehaltiger Biomasse wie zum Beispiel Weizenstroh als Monosubstrat bereit. Dabei wird besonders durch die Einstellung des Natriumgehalts, gegebenenfalls durch Dosierung von Natronlauge oder Natriumsalzen wie zum Beispiel Natriumhydrogencarbonat, im gesamten Fermentationssystem von etwa 100mg/l bis 5.000mg/l, vorzugsweise von 400mg/l bis 2.000mg/l, die Biogasbildung erheblich verbessert. Ebenso wird durch die Einstellung des Gehalts an Ammoniumstickstoff, gegebenenfalls durch Dosierung von zum Beispiel Ammoniumsalzen wie Ammoniumhydrogencarbonat oder anderen stickstoffhaltigen Stoffen, im gesamten Fermentationssystem von etwa 200mg/l bis 2.000mg/l, vorzugsweise 500mg/l bis 1.200mg/l, eine Stabilisierung und weitere Verbesserung der Biogasbildung erzielt.

Durch den hohen Rückführungsgrad von Prozesswasser aus der Fest-Flüssig-Trennung der Gärrestbehandlung zum Anmischen kann der Bedarf an zusätzlichen Makro- und Mikronährstoffen, insbesondere von Natrium und Stickstoff, deutlich reduziert oder sogar vollständig vermieden werden.

Zudem besteht die Möglichkeit den Trockensubstanzgehalt von anfallenden Gärresten durch eine aerobe Fermentation mit Pilzen weiter zu verringern und bei nochmaliger Vergärung des derart behandelten Gärrests zusätzliches Biogas zu produzieren und schließlich durch den damit einhergehenden weiteren Trockensubstanzabbau die Prozesswasserrückführung zu erhöhen.

Durch eine Kombination der zuvor genannten Maßnahmen ist der mit dem anaeroben Abbau zu Biogas einhergehende Trockensubstanzabbau derart hoch, dass eine Rückführung von Prozesswasser aus der Fest-Flüssig-Trennung den Flüssigkeitsbedarf beim Einmischen fast vollständig oder vollständig decken kann.

In einer nicht erfindungsgemäßen Ausführungsform ist das Verfahren zur Herstellung von Biogas aus lignocellulosehaltiger Biomasse, vorzugsweise aus Stroh, als Verfahren mit nur einer Fermentationsstufe ausgestaltet, welches die folgenden Schritte umfasst:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, insbesondere mit Prozesswasser und/oder unter Zuhilfenahme von Dampf;
b) Erhitzung der lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss
c) anaerobe Fermentation der thermisch behandelten lignocellulosehaltigen Biomasse mithilfe von Mikroorganismen und Gewinnung des entstehenden Biogases.

In einer nicht erfindungsgemäßen alternativen Ausführungsform ist das Verfahren zur Herstellung von Biogas aus lignocellulosehaltiger Biomasse, vorzugsweise aus Stroh, als Verfahren mit zwei Fermentationsstufen ausgestaltet, welches folgende Schritte umfaßt:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, insbesondere mit Prozesswasser und/oder unter Zuhilfenahme von Dampf;
a1) anaerobe Fermentation der angemischten lignocellulosehaltigen Biomasse mithilfe von Mikroorganismen und Gewinnung des entstehenden Biogases;
b) Erhitzung der lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss
c) anaerobe Fermentation der thermisch behandelten lignocellulosehaltigen Biomasse mithilfe von Mikroorganismen und Gewinnung des entstehenden Biogases.

Die Ausgestaltung des Verfahrens als Verfahren mit zwei anaeroben Fermentationsstufen führt zu besonders hohen Ausbeuten an Biogas.

Aus einer nicht erfindungsgemäßen Ausführungsform des Verfahrens beinhaltet das Verfahren einen im Gegenstromprinzip arbeitenden Wärmeaustausch zwischen der angemischten lignocellulosehaltigen Biomasse aus Schritt a) und der thermischen behandelten lignocellulosehaltigen Biomasse aus Schritt b), um eine energieeffiziente Erreichung der Arbeitstemperatur des thermischen Aufschlusses zu ermöglichen. Eine nicht erfindungsgemäße Ausführungsform des Verfahrens umfasst daher folgende Schritte:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, insbesondere mit Prozesswasser und/oder unter Zuhilfenahme von Dampf;
a2) Durchführung eines im Gegenstromprinzip arbeitenden Wärmeaustausches zwischen der in Schritt a) erhaltenen lignocellulosehaltigen Biomasse, insbesondere nach vorheriger Einstellung des Trockensubstanzgehaltes auf 7-20 Gew.-%, und der in Schritt b) erhaltenen thermisch behandelten lignocellulosehaltigen Biomasse.
b) Erhitzung der lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss
c) anaerobe Fermentation der thermisch behandelten lignocellulosehaltigen Biomasse mithilfe von Mikroorganismen und Gewinnung des entstehenden Biogases.

Vorzugsweise erfolgt dieser im Gegenstromprinzip arbeitende Wärmeaustausch so, dass eine nachfolgende Erhitzung um weniger als 20°K eine energieeffiziente Erreichung der Arbeitstemperatur des thermischen Aufschlusses in Schritt b) ermöglicht.

In einer nicht erfindungsgemäßen alternativen Ausführungsform ist das Verfahren so ausgestaltet, dass es die folgenden Schritte umfasst:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, insbesondere mit Prozesswasser und/oder unter Zuhilfenahme von Dampf;
a1) anaerobe Fermentation der angemischten lignocellulosehaltigen Biomasse mithilfe von Mikroorganismen und Gewinnung des entstehenden Biogases
a2) Durchführung eines im Gegenstromprinzip arbeitenden Wärmeaustausches zwischen der in Schritt a) erhaltenen lignocellulosehaltigen Biomasse, insbesondere nach vorheriger Einstellung des Trockensubstanzgehaltes auf 7-20 Gew.-%, und der in Schritt b) erhaltenen thermisch behandelten lignocellulosehaltigen Biomasse.
b) Erhitzung der lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss
c) anaerobe Fermentation der thermisch behandelten lignocellulosehaltigen Biomasse mithilfe von Mikroorganismen und Gewinnung des entstehenden Biogases.

Vorzugsweise erfolgt der im Gegenstromprinzip arbeitende Wärmeaustausch so, dass eine nachfolgende Erhitzung um weniger als 20°K eine energieeffiziente Erreichung der Arbeitstemperatur des thermischen Aufschlusses in Schritt b) ermöglicht.

Unter lignocellulosehaltiger Biomasse soll im Sinne der vorliegenden Erfindung insbesondere eine Biomasse verstanden werden, die als wesentliche Bestandteile Lignin, Hemicellulose und Cellulose aufweist, insbesondere in Form von Stroh, insbesondere Getreidestroh, Holz und/oder Gräsern.

Nachfolgend wird das integrierte Lösungsprinzip zur Herstellung von Biogas aus lignocellulosehaltiger Biomasse am Beispiel von Getreidestroh, nachfolgend Stroh genannt, beschrieben, ohne dass damit eine Einschränkung vorgenommen werden soll. Die technischen Mittel und Wirkungsprinzipien sind für den Fachmann selbstverständlich leicht auf andere Arten von lignocellulosehaltiger Biomasse wie zum Beispiel Gräser, Hölzer oder Mischsubstrate übertrag- und anwendbar. Deren Übertragung wird somit nicht expliziert erläutert. Es ist auch selbstverständlich, dass sich das beschriebene Verfahren und die Anlage auch zusammen mit Anteilen aus nicht lignocellulosehaltiger Biomasse wie zum Beispiel Gülle grundsätzlich zur Biogaserzeugung eignen.

Die in der Beschreibung aufgeführten Prozentangaben sind, falls nicht explizit anders aufgeführt, als Massenanteil zu interpretieren.

Zunächst wird das Stroh in der Regel in Quaderballen mit einem Trockensubstanzgehalt von 80% bis 95% angeliefert und von Fremdstoffen befreit. Die mechanische Zerkleinerung des Strohs erfolgt aus energetischen Gründen mehrstufig. Zunächst wird der Strohballen mechanisch aufgelöst und das Stroh bei Bedarf auf eine Partikelgröße kleiner 200mm vorzerkleinert. Die anschließende Zerkleinerung auf eine für den Prozess besonders günstige Partikelgröße von vorzugsweise 1mm bis 10mm kann zum Beispiel in einer Hammermühle, einem Häcksler oder einem Schredder erfolgen. In besonderen Fällen kann die Zerkleinerung zu Mehl mit einer Partikelgröße <200µm sinnvoll sein, um durch diesen physikalischmechanischen Aufschluss die Substratoberfläche signifikant zu erhöhen.

Anschließend wird das Stroh zur Zerstörung der Wachsschicht einer thermischen Behandlung unterzogen. Das zerkleinerte Stroh wird in einem Dämpfer mit integriertem Mischer mit Heißwasser und/oder Dampf beaufschlagt, so dass das derart angefeuchtete Stroh eine Temperatur von 60°C bis 100°C, vorzugsweise 70°C bis 85°C, erreicht. Dadurch wird die Wachsschicht der Lignocellulosefasern des Strohs ausreichend zerstört, wodurch die Wasseraufnahme verbessert und dadurch die Bildung von Schwimmschichten bei der späteren Fermentation unterbunden wird. Dadurch ist eine signifikante Verringerung der notwendigen Rührleistung und damit eine Energieeinsparung während der Fermentation möglich. Das angefeuchtete Stroh wird nach einer Aufenthaltszeit von 5min bis 30min, bevorzugt 5min bis 15min mit einem Trockensubstanzgehalt von 20% bis 60%, bevorzugt ca. 30% ausgetragen. Wird in diesem Teilprozess Heißwasser eingesetzt, so kann dieses vorzugsweise prozessintern gewonnene Flüssigkeit sein, welche direkt oder nach Erwärmung, insbesondere nach Erwärmung mit prozessintern bereitstehender Abwärme, dem Dämpfer zugeführt wird. Darüber hinaus ist eine Dosierung von Enzymen wie zum Beispiel ligninmodifizierenden Enzymen, Glucanasen, Hemicellulasen und Pektinasen in den Dämpfer möglich, um einen ersten Abbau der lignocellulosehaltigen Biomasse zu erwirken.

Nachdem das Stroh angefeuchtet wurde, besteht die Möglichkeit einer aeroben biologischen Behandlung mit Pilzen oder alternativ mit Enzymen zum gezielten Ligninabbau und damit einhergehender besserer Verfügbarkeit von Cellulose und Hemicellulose in der anschließenden anaeroben Fermentation. Vorteilhaft für die biologische Behandlung ist der pasteurisierende Effekt der vorherigen thermischen Behandlung. Werden Pilze zugegeben geschieht dies unter Einstellung, in der Regel unter Absenkung des pH-Wertes auf ca. 3,5 bis 8, besonders auf 4 bis 6, zum Beispiel durch Dosierung von Säuren. Als Lignin spaltende oder abbauende Pilze eigenen sich insbesondere Basidiomyceten-Stämme, Pleurotus ostreatus, Phellinus torulosus, Coriolus hirsutus, Trametes hirsuta, Cyathus helenae, Cyathus olla, Coprinus comatus, Cyathus limbatus, Polyporus adustus, Chyrosporium lignorum und Peniophora crema und deren speziell für einen primären Ligninaufschluss/-abbau statt Cellulose- und Hemicelluloseabbau angepassten Stämme oder deren Enzyme. Die Dosierung der Pilze kann zum Beispiel mit wässrigen Anzuchtkulturen in dafür geeigneten Behältnissen erfolgen. Der aerobe Abbau erfolgt unter Zuführung von geeigneten Mengen Sauerstoff, vorzugsweise in Form von Luft, insbesondere von feuchter Luft. Als Apparate können insbesondere Räume sowie aerobe Behälter mit Gaszufuhr in Bodennähe oder belüftete Schläuche mit großen Querschnitten eingesetzt werden. Entsprechende Schläuche, wie sie zum Beispiel aus der Silierung bekannt sind, können auf Freiflächen oder in Hallen gelagert werden und mit am Boden liegenden Gaszufuhrröhren/-spießen mit Gasaustrittsöffnungen oder im Schlauchboden befestigten Gaseinlässen zum Beispiel in Form von Düsen sowie Auslassöffnungen auf der Schlauchoberseite vorzugsweise als Überdruckventil ausgeführt werden, um eine effektive Frischgaszirkulation für das Pilzwachstum zu gewährleisten. Die Temperatur sollte dabei zwischen ca. 10°C und 40°C liegen, bevorzugt zwischen 15°C bis 35°C. Als Verweilzeit werden ca. 1-4 Wochen, besonders ca. 1-2 Wochen bevorzugt, für den Fall, dass mit den Pilzen und deren Enzymen vorrangig eine für die Biogaserzeugung unterstützende Freilegung von Cellulose und Hemicellulose anstatt des Ligninabbaus erreicht werden soll. Diese Werte sind als Richtwerte zu betrachten und abhängig von der jeweiligen Pilzart, des Substrates und dessen Zerkleinerungsgrad sowie eingesetzten Zuschlagstoffen, insbesondere Enzymen. Durch eine Steuerung des Sauerstoff- und Kohlendioxidgehaltes, gegebenenfalls der Zugabe von den Cellulose- und/oder Hemicelluloseabbau hemmenden Inhibitoren kann der Verlust leicht vergärbaren Substrats verhindert und/oder das Wachstum von Konkurrenzmikroorganismen verlangsamt werden.

Alternativ oder additiv können dem angefeuchteten Stroh auch Enzyme für einen enzymatischen Aufschluss zudosiert werden. Hierfür eignen sich Enzyme wie zum Beispiel ligninmodifizierende Enzyme, Ligninasen, insbesondere Laccasen sowie Glucanasen und Pektinasen. Beim Einsatz von Enzymen, sollte der Trockensubstanzgehalt des feuchten Strohs so gewählt werden, dass freies Wasser anwesend ist. Die Wirkdauer der Enzyme beträgt zwischen 1-24h, vorzugsweise 2-6h, bei einer Temperatur zwischen 5-80°C, bevorzugt 20-50°C und einem pH-Wert zwischen 2-10, bevorzugt 5-8. Für ein optimales Wirken der Enzyme ist ein kontinuierliches oder diskontinuierliches Mischen des Substrates zum Beispiel in einem Rührbehälter sinnvoll.

Das befeuchtete, gegebenenfalls auch biologisch und/oder enzymatisch behandelte, Stroh wird mit Wasser, vorzugsweise mit rezirkuliertem Prozesswasser, zu einer pumpfähigen, vergärbaren Suspension angemischt. Die Anmischung des feuchten Strohs mit Wasser kann in einem Mischer, zum Beispiel aber auch in einer Rachentrichterpumpe erfolgen. Dabei hat sich für die folgende Nassvergärung dieser Suspension ein Trockensubstanzgehalt von 5% bis 30%, bevorzugt 10% bis 18%, als besonders geeignet erwiesen. Durch Einstellung der Temperatur des rezirkulierten Prozesswassers kann die Temperatur der Suspension so eingestellt werden, dass die Fermentationstemperatur der sich anschließenden anaeroben Fermentation im beabsichtigten optimalen Bereich gehalten wird. Alternativ kann die Temperierung der Suspension und damit der nachfolgenden Fermentation auch über eine indirekte Wärmeübertragung in einem Wärmetauscher, zum Beispiel einem Plattenwärmeübertrager oder einem Rohrbündelapparat, gegebenenfalls unter Nutzung von Abwärmeströmen oder Kühlwasser erfolgen.

Es kann in einigen Fällen sinnvoll sein, die in der Suspension eingemischte lignocellulosehaltige Biomasse weiter zu zerkleinern, insbesondere wenn die mittlere Substratpartikelgröße größer als 2mm ist. Dies kann beispielsweise mit einer Nasszerkleinerung durch eine Nassmühle oder Schneidräder im Rohrtransportsystem oder Dispergierer erreicht werden. Die Nasszerkleinerung kann bei Bedarf auch mehrstufig ausgeführt sein. Sie kann in günstiger Weise direkt nach der Anmischung, aber auch nach dem Wärmeübertrager unmittelbar vor der anaeroben Fermentation installiert werden.

Die erzeugte Suspension wird zusammen mit Stickstoffquellen wie zum Beispiel Ammoniumsalz und/oder mit Zuschlagsstoffen, insbesondere Makro- und Mikronährstoffen wie Natrium- und/oder Phosphor- und/oder Zinkverbindungen, einer anaeroben Fermentation in einem oder mehreren Fermentern unterzogen. Üblicherweise erfolgt die Fermentation kontinuierlich oder bei getakteter Zuführung von Substrat in mehrere Fermenter zumindest quasikontinuierlich. Der oder die Fermenter sollten mit mindestens einem Rührwerk ausgestattet sein, um eine ausreichende Homogenisierung des Fermenterinhalts zu gewährleisten und den Stofftransport zu verbessern.

Überraschenderweise wurde in Fermentationsversuchen festgestellt, dass eine Einstellung des Ammoniumgehalts im gesamten Fermentationssystem auf 200mg/l bis 2.000mg/l, vorzugsweise 500mg/l bis 1.200mg/l, den Abbauprozess stabilisiert und die Biogasbildung erheblich verbessert.

Darüber hinaus wurde in Langzeitabbauversuchen überraschenderweise festgestellt, dass durch die Einstellung des Natriumgehalts im gesamten Fermentationssystem auf etwa 100mg/l bis 5.000mg/l, vorzugsweise auf 400mg/l bis 2.000mg/l, die Biogasbildung erheblich gesteigert wird. Die Einstellung des Natriumgehalts kann durch diverse Natriumcarbonate, Natronlauge oder auch andere natriumhaltige organische Stoffe oder Reststoffe erfolgen. Dabei muss die Dosierung des natriumhaltigen Stoffes nicht zwangsläufig direkt in die Suspension oder direkt in den Fermenter erfolgen, sondern vielmehr kann der optimale Natriumgehalt auch indirekt über das rezirkulierte Prozesswasser eingestellt werden.

Des Weiteren kann sich die Dosierung von Makro- und Mikronährstoffen wie zum Beispiel Kalium, Calcium, Magnesium, drei- und zweiwertigem Eisen und Spurenelementen wie zum Beispiel Kobalt, Kupfer, Molybdän, Nickel, Selen und Wolfram sowie deren Verbindungen und von Vitaminen positiv auf den Stoffwechsel der Mikroorganismen und damit auf die Biogasausbeute auswirken.

Als Mikroorganismen kommen insbesondere solche zum Einsatz, die eine Hydrolyse, Acidogenese, Acetogenese und Methanogenese vornehmen. Die Verweilzeit der anaeroben Fermentation wird so gewählt, dass sich im Fermenter eine stabile Mikroorganismenpopulation etabliert. Vorteilhaft ist bei einem mesophilen oder thermophilen Betrieb eine Verweilzeit von 10 bis 50 Tagen, vorzugsweise 15 bis 30 Tage. Das erzeugte Biogas wird gesammelt und zur weiteren Aufbereitung abgeführt.

Nachdem die lignocellulosehaltige Biomasse in der ersten Fermentationsstufe zumindest teilweise zu Biogas vergoren wurde, wird der feststoffhaltige Ablauf der Fermentation einem kontinuierlich arbeitenden thermischen Aufschluss zugeführt. Nicht erfindungsgemäß besteht in einer anderen Prozessführung auch die Möglichkeit die vergärbare Suspension ohne vorherige anaerobe Fermentation direkt dem thermischen Aufschluss zuzuführen und die aufgeschlossene Suspension einer anaeroben Fermentation zuzuführen.

Überraschenderweise wurde in Versuchen festgestellt, dass ein solcher Aufschluss auch ohne plötzliche Druckentspannung den Faserverbund der Lignocellulosestruktur soweit zerstört, dass zuvor nicht abbaubare organische Komponenten, insbesondere weitere Cellulose und Hemicellulose, in technisch relevanten Mengen für den anaeroben Abbau zugänglich gemacht werden. Dadurch wird die Biogasausbeute signifikant gesteigert. Der pumpfähige Ablauf wird dafür mit einer geeigneten Pumpe, wie zum Beispiel einer Exzenterschneckenpumpe, in das unter Druck stehende Aufschlusssystem gefördert, indem zunächst eine zweistufige Erwärmung, nachfolgend ein thermischer Aufschluss sowie schließlich eine ein- oder zweistufige Abkühlung erfolgt.

Die erste Stufe der Erwärmung des Ablaufs erfolgt, indem indirekt Wärme von heißem, bereits aufgeschlossenem Ablauf derart übertragen wird, das die Temperatur des dann erwärmten Ablaufs maximal 20K, bevorzugt maximal 10K, unterhalb der beabsichtigten Aufschlusstemperatur liegt. Die restliche Erwärmung erfolgt in der zweiten Stufe durch indirekte Wärmeübertragung von einem Wärmeträgermedium, wie zum Beispiel Dampf, Wasser oder Thermalöl, oder durch Direktdampf auf die beabsichtigte Aufschlusstemperatur. Der thermische Aufschluss findet günstigerweise bei 130°C bis 200°C, vorzugsweise bei 150°C bis 170°C statt. Durch einen als Druckbehälter ausgeführten Aufschlussreaktor wird eine mittlere Verweilzeit des Ablaufs bei dieser Aufschlusstemperatur von 5min bis 60min, vorzugsweise von 10min bis 30min realisiert. Der Aufschlussreaktor kann zur Homogenisierung und zur Vermeidung von Kurzschlussströmungen mit einem Rührwerk oder anderen Mischaggregaten ausgestattet sein. Nachfolgend wird der aufgeschlossene Ablauf indirekt am zu erwärmenden Ablauf abgekühlt und gegebenenfalls in einem zweiten Wärmeübertrager weiter indirekt oder direkt abgekühlt, um schließlich einem weiteren anaeroben Abbau zu Biogas zugeführt zu werden. Als Wärmeübertrager können zum Beispiel druckfeste Plattenwärmeübertrager oder Rohrbündelapparate benutzt werden.

Vorteilhaft bei diesem Konzept ist die Verringerung des thermisch zu behandelnden Stoffstroms gegenüber eines direkten thermischen Aufschlusses des Strohs vor der ersten Fermentation. In Verbindung mit dem kontinuierlich arbeitenden Aufschlusssystem und der beschriebenen internen Wärmeübertragung wird der Energiebedarf gegenüber herkömmlichen Aufschlussverfahren zur Behandlung lignocellulosehaltiger Biomasse signifikant verringert. Durch eine Erhöhung des TS-Gehalts des Ablaufs durch partielle Fest-Flüssig-Trennung und Anreicherung des übrigen Ablaufs mit dem gewonnenen Feststoff kann die energetische Effizienz des Aufschlusssystems weiter verbessert werden. Dabei kann die Fest-Flüssig-Trennung zum Beispiel durch Dekanter, Zentrifugen oder Pressen realisiert werden. Günstigerweise wird die erste Fermentation und/oder die Fest-Flüssig-Trennung so betrieben, dass der TS-Gehalt des thermisch zu behandelnden Ablaufs im Bereich von 7% bis 20%, besonders bevorzugt im Bereich von 9% bis 13%, liegt. Eine in der Fest-Flüssig-Trennung anfallende Flüssigphase wird, gegebenenfalls nach einer Zwischenspeicherung, prozessintern, bevorzugt zur Anmischung von befeuchtetem Stroh rezirkuliert.

Der anaerobe Abbau des thermisch behandelten Ablaufs wird durch Rückführung in die erste Fermentation zusammen mit frischem Substrat bzw. bevorzugt durch Zuführung in eine weitere, zweite Fermentation realisiert. Letztere kann mesophil als auch thermophil betrieben werden, wobei die Temperatur durch den Zustrom an behandeltem Ablauf in jedem Fall aufrechterhalten werden kann. Energetische Vorteile können sich bei der Realisierung einer thermophilen Vergärung ergeben, da die Abwärme aus dem Aufschluss zur Temperierung genutzt und im weiteren Prozessverlauf Prozessflüssigkeit mit einer höheren Temperatur in die erste Fermentation rezirkuliert werden kann. Günstigerweise wird auch die zweite Fermentation derart betrieben, dass die Mikroorganismenpopulation in einem produktiven, stationären Zustand gehalten wird. Dies ist bei einer Verweilzeit von 5 bis 15 Tagen gewährleistet. Die technische Umsetzung der zweiten Fermentation kann in gleicher Weise wie bei der ersten Fermentation erfolgen.

Durch Rückführung von aufgeschlossenem Ablauf zurück in die erste Fermentation (einstufige Fermentation) muss ein Teil des bei der Fest-Flüssig-Trennung gewonnenen Feststoffs aus dem System ausgeschleust werden. Über diesen Auslass werden das nicht abbaubare Lignin sowie mineralische Komponenten ausgetragen. Dieser Stoffstrom steht als Grundstoff für eine spätere stoffliche Nutzung des Lignins und/oder direkt oder nach weiterer Behandlung als organischer Dünger für die Landwirtschaft zur Verfügung.

Der Ablauf aus dem zweiten Fermenter kann als organischer Dünger verwendet werden oder wird bevorzugt durch eine Fest-Flüssig-Trennung, in eine flüssige und eine feststoffhaltige Phase getrennt. Die feststoffhaltige Phase kann zur Erhöhung der Raumbelastung und Verlängerung der Feststoffverweilzeit und damit zur Steigerung der Biogasausbeute anteilig in die zweite Fermentation zurückgeführt werden. Mit einer weiteren Erhöhung der Biogasausbeute verringert sich die aus dem Gesamtverfahren auszuschleusende Trockensubstanz weiter, wodurch Kosteneinsparungen im Bereich der Logistik eintreten.

Der analog zur einstufigen Fermentation auszuschleusende Anteil der feststoffhaltigen Phase kann zum Beispiel als Ligninquelle oder auch als organischer Dünger verwendet werden. Bei Verwendung als organischer Dünger kann sowohl die Humus- als auch die Nährstoffversorgung, insbesondere die Versorgung mit Kalium, Stickstoff und Phosphor, der landwirtschaftlichen Flächen bzw. des darauf stattfindenden Pflanzenbaus wesentlich verbessert werden. Wird die anaerob nicht abbaubare organische Substanz (vor allem das Lignin) auf die entsprechenden Anbauflächen, von denen zuvor das Stroh für die Biogasgewinnung vollständig entnommen wurde, ausgebracht, so kann die Humusbilanz vollständig ausgeglichen werden.

Die bei der Fest-Flüssig-Trennung gewonnene Flüssigphase wird prozessintern, vorzugsweise in die Anmischung des Strohs vor der ersten Fermentation, rezirkuliert. Dadurch kann der Wasserbedarf signifikant verringert oder sogar vollständig prozessintern gedeckt werden. Dies gilt insbesondere auch für überwiegend feststoffhaltige Substrate wie zum Beispiel Stroh, da durch das vorgeschlagene Verfahren die Biogasausbeute und damit einhergehend der Trockensubstanzabbau signifikant gesteigert wird, wodurch die Flüssigkeitsverluste durch die Biogasbildung selbst bzw. durch die Ausschleusung der feststoffhaltigen Phase nahezu vollständig oder vollständig durch die Feuchtigkeit des Substrats ausgeglichen werden können. Darüber hinaus bewirkt die Rezirkulation der Flüssigphase eine Rückführung von Makro- und Mikronährstoffen wie zum Beispiel Stickstoff und Natrium sowie teilweise auch von aktiven Mikroorganismen selbst. Dies führt einerseits zu einer Stabilisierung des Prozesses sowie andererseits zur Verringerung der Dosierung von Zuschlagstoffen, wodurch die Wirtschaftlichkeit des gesamten Verfahrens verbessert wird.

Zur Gewinnung von in der flüssigen Phase enthaltenen Wertstoffen wie zum Beispiel Kaliumverbindungen oder gelösten Ligninkomponenten bzw. zur Abtrennung von Störstoffen kann eine Behandlung der flüssigen Phase vor der Rezirkulation vorgenommen werden. Entsprechende Behandlungsverfahren können zum Beispiel Membranverfahren, Filter und/oder selektive Fällungen, eine Eindampfung oder eine aerobe Behandlung sein.

Zusätzlich besteht die Möglichkeit den zweiten Ablauf bzw. die enthaltene feststoffhaltige Phase nach einer vollständigen oder anteiligen Phasentrennung einer biologischen Nachbehandlung mit Lignin abbauenden Pilzen analog der vorher beschriebenen Behandlung des Strohs vor der ersten Fermentation zu unterziehen. Dadurch könnten weitere Feststoffe abgebaut werden, wodurch der abzutransportierende Feststoff (Dünger) mengenmäßig weiter reduziert wird und noch mehr Prozessflüssigkeit generiert werden kann. Zudem ist bei Rückführung des derart behandelten Ablaufs bzw. der derart behandelten feststoffhaltigen Phase in die Fermentation ein weiterer Biogasertrag möglich, wodurch die Effizienz des Gesamtverfahrens erhöht wird.

Während sich alle bisher bekannten Verfahren und/oder Anlagen zur Vergärung von lignocellulosehaltiger Biomasse zu Biogas auf Grund ihrer geringen Wirtschaftlichkeit nicht etablieren konnten, ist die Erzeugung von Biogas aus ebensolcher lignocellulosehaltiger Biomasse als Hauptsubstrat bzw. sogar als Monosubstrat mit der beschriebenen Lösung wirtschaftlich möglich.

Die vorgeschlagene Lösung verbessert das Verhältnis von Prozessenergiebedarf zum Biogasertrag gegenüber bekannten Verfahren signifikant und führt durch die optimierte Prozesswassergewinnung und -rückführung zu wesentlichen Verbesserungen im Hinblick auf den Wasserbedarf und Hilfsstoffeinsatz und damit zu deutlichen ökonomischen Verbesserungen.

### KURZBESCHREIBUNG DER ZEICHNUNGSFIGUREN

Fig. 1 zeigt eine schematische Darstellung einer Variante eines einstufigen Verfahrens zur Herstellung von Biogas aus lignocellulosehaltiger Biomasse (nicht gemäß der Erfindung);
Fig. 2 zeigt eine schematische Darstellung einer Ausführungsform eines mehrstufigen Verfahrens zur Herstellung von Biogas aus lignocellulosehaltiger Biomasse gemäß der Erfindung;

### DETAILLIERTE BESCHREIBUNG BEVORZUGTER AUSFÜHRUNGSFORMEN

Beispiele sind in den Zeichnungen dargestellt und werden nachfolgend näher beschrieben.

### Beispiel 1

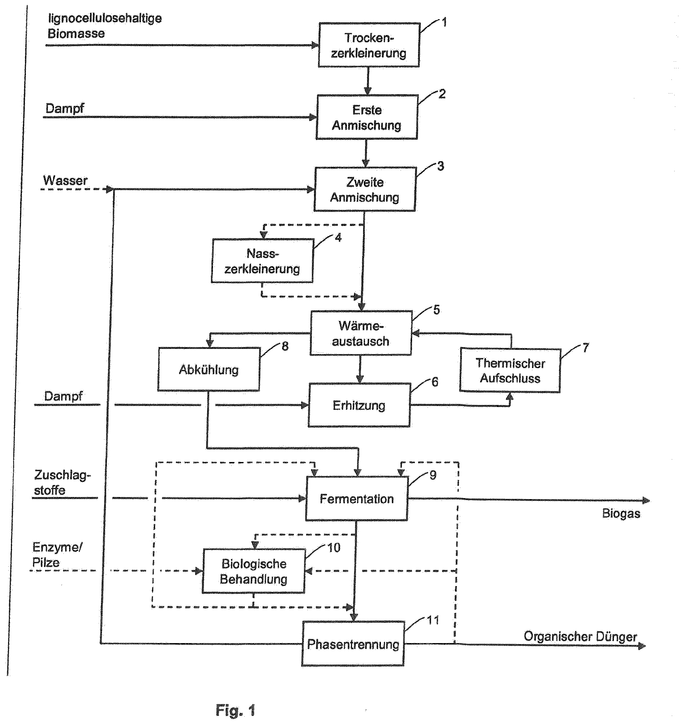

Fig. 1 zeigt ein schematisches Beispiel eines einstufigen Verfahrens zur Herstellung von Biogas aus lignocellulosehaltiger Biomasse wie zum Beispiel Stroh oder Holz (nicht gemäß der Erfindung).

Die lose oder gepresste lignocellulosehaltige Biomasse wird gegebenenfalls nach einer Reinigung von Steinen und sonstigen Fremdkörpern zunächst der Trockenzerkleinerung 1 zugeführt. In einem Schredder, einem Häcksler oder einer Hammermühle wird die lignocellulosehaltige Biomasse auf eine Substratpartikelgröße kleiner 200mm, bevorzugt kleiner 20mm, bei Stroh besonders bevorzugt auf eine Substratpartikelgröße zwischen 1mm und 10mm zerkleinert. Alternativ oder zusätzlich kann eine Bearbeitung in einem Nasszerkleinerer stattfinden.

Anschließend wird das zerkleinerte Substrat zum Beispiel pneumatisch in die erste Anmischung 2 gefördert. Die erste Anmischung 2 ist als Dämpfer, Mischer, Rührer oder Umwälzer ausgeführt. In diesem Apparat wird das zerkleinerte Substrat mit Heißwasser und/oder Dampf beaufschlagt, um es auf 60-100°C, bevorzugt auf 70-85°C, für eine Dauer von 0,5min bis 30min, bevorzugt 5min bis 15min zu erwärmen. Durch die erhöhten Temperaturen wird das Entgasen des Substrats erwirkt und die Aufnahmefähigkeit von Wasser verbessert, indem insbesondere eine Auflösung der Wachsschicht einsetzt. Durch die Wasseraufnahme des Substrats steigt die Dichte der Partikel, wodurch eine Schwimmschichtbildung in der Fermentation 9 reduziert oder sogar vollständig verhindert wird. In der ersten Anmischung 2 wird ein befeuchtetes Substrat mit einem TS-Gehalt zwischen 20% und 60%, bevorzugt ca. 30% generiert.

Das durch die erste Anmischung 2 befeuchtete Substrat wird in der zweiten Anmischung 3 zusammen mit Wasser zu einer Suspension mit einem TS-Gehalt zwischen 5% und 30%, bevorzugt zwischen 10% und 18% angemischt. Für das Anmischen werden bevorzugt Prozesswässer aus der Phasentrennung 11 verwendet, wodurch der Frischwasserbedarf fast vollständig oder sogar vollständig gedeckt werden kann. Zudem werden mit dem zurückgeführten Prozesswasser Nährstoffe, Spurenelemente und Mikroorganismen rezirkuliert.

In einigen Verfahrensformen kann es zudem sinnvoll sein, das in der Suspension eingemischte Substrat weiter zu zerkleinern, insbesondere wenn die mittlere Substratpartikelgröße größer als 2 mm ist. Dies kann mit einer Nasszerkleinerung 4 beispielsweise durch eine Nassmühle erreicht werden.

Die pumpfähige Suspension wird einem kontinuierlich arbeitenden thermischen Aufschluss 7 zugeführt, um den Faserverbund der Lignocellulosestruktur aufzubrechen und auch schwerer abbaubare Bestandteile für einen effizienten anaeroben Abbau zugänglich zu machen. Die Förderung in den unter Druck arbeitenden thermischen Aufschluss 7 kann zum Beispiel mit einer Exzenterschneckenpumpe erfolgen. Auch der Wärmetauscher 5 und die Erhitzung 6 sowie die Abkühlung 8 werden unter einem solchen Druck betrieben, dass aus der Suspension kein Wasserdampf entsteht. Der thermische Aufschluss 7 erfolgt in wässriger Phase bei einer Temperatur von 130°C bis 200°C, bevorzugt bei einer Temperatur von 150°C bis 170°C. Um den energetischen Aufwand des thermischen Aufschlusses zu minimieren, erfolgt zunächst eine indirekte Aufwärmung der Suspension in einem bevorzugt im Gegenstrom arbeitenden Wärmetauschers 5. Dabei wird Wärme von der bereits thermisch behandelten heißen Suspension auf die noch zu behandelnde Suspension übertragen. Der Wärmetauscher 5 überträgt dabei so viel Wärme, dass die Temperatur der erwärmten Suspension maximal 20K, bevorzugt maximal 10K unter der gewünschten Aufschlusstemperatur liegt. Im Anschluss erfolgt die weitere Erwärmung bis auf die Aufschlusstemperatur in der Erhitzung 6 mit Dampf. Dies kann indirekt zum Beispiel in einem Plattenwärmeübertrager oder durch Eindüsung von Direktdampf erfolgen. Schließlich strömt die heiße Suspension in den Aufschlussreaktor (thermischer Aufschluss 7). In diesem isolierten Druckbehälter wird eine Verweilzeit von 5min bis 60min, vorzugsweise 10min bis 30min bei der entsprechenden Aufschlusstemperatur realisiert. Zur Verbesserung des Strömungsverhaltens kann der Aufschlussreaktor mit einem Rührwerk bzw. einer anderen Misch- oder Fördereinrichtung ausgestattet sein.

Die heiße aufgeschlossene Suspension passiert erneut den Wärmeaustausch 5, um die Wärme an die noch zu behandelnde Suspension abzugeben. Danach wird die aufgeschlossene Suspension in der Abkühlung 8, welche zum Beispiel mit Kühlwasser arbeitet, so temperiert, dass die Temperatur der Fermentation 9 in einem für den meso- oder thermophilen anaeroben Abbau geeigneten Temperaturbereich gehalten wird.

Die erzeugte Suspension wird gegebenenfalls zusammen mit Zuschlagstoffen einer anaeroben Fermentation 9 zugeführt. Zuschlagstoffe können Stickstoffquellen beispielsweise in Form von Ammoniumhydrogencarbonat und/oder Makro- und Mikronährstoffen wie zum Beispiel Natrium- und/oder Phosphor- und/oder Zinkverbindungen und/oder kommerzielle Spurenelementlösungen sein. Die Fermentation 9 kann aus einem oder mehreren parallel betriebenen Fermentern bestehen, welche vorzugsweise mit einem Rührwerk ausgestattet sind und günstigerweise durch eine getaktete Substratzufuhr quasikontinuierlich betrieben werden. Durch die Vergärung im Anschluss an den thermischen Aufschluss wird die Gesamtausbeute an Biogas innerhalb wirtschaftlicher Verweilzeiten deutlich gesteigert. Die Verweilzeit der mesophilen oder thermophilen anaeroben Fermentation 9 beträgt vorzugsweise 10 bis 30 Tage. Das kontinuierlich erzeugte Biogas wird im oberen Teil des Fermenters bzw. der Fermenter abgezogen und über eine Sammelleitung zur weiteren Aufbereitung geführt.

Der Ablauf aus der Fermentation 9 enthält nur noch sehr schwer abbaubare Faserbestandteile, welche zu großen Teilen aus Lignin bestehen. Durch den Mineraliengehalt und die noch enthaltenen organischen Bestandteile kann der Ablauf sehr gut als organischer Dünger eingesetzt werden. Vorzugsweise wird der Ablauf jedoch zunächst einer Phasentrennung 11 zugeführt und in eine flüssige und eine feststoffhaltige Phase getrennt. Diese Fest-Flüssig-Trennung kann zum Beispiel durch einen Dekanter realisiert werden. In diesem Fall wird eine stichfeste, gut lagerfähige feste Phase mit kaum vorhandenem oder gar keinem freien Wasser erzeugt. Die feststoffhaltige Phase kann zur weiteren Ausbeutesteigerung anteilig in die Fermentation zurückgeführt werden. Dadurch wird eine höhere Feststoffverweilzeit erreicht und die Biogasausbeute erhöht. Der überschüssige Anteil der feststoffhaltigen Phase kann gegebenenfalls nach einer Zwischenlagerung als organischer Dünger in der Landwirtschaft verwendet werden. Dadurch kann die Humusbilanz und Mineralstoffzufuhr auf die Agrarflächen wesentlich verbessert und eine nachhaltige Bewirtschaftung sichergestellt werden.

Die in der Phasentrennung 11 erzeugte flüssige Phase wird als Prozesswasser gegebenenfalls nach einer Zwischenlagerung in die zweite Anmischung 3 zurückgeführt. Durch den effektiven Abbau des Substrats zu Biogas wird gleichzeitig so viel TS abgebaut, dass in der Phasentrennung 11 viel Prozesswasser generiert wird, wodurch der Wasserbedarf des Gesamtprozesses der Biogaserzeugung aus lignocellulosehaltiger Biomasse besonders gering ist. Die rezirkulierte Menge Prozesswasser kann so hoch sein, dass gar kein Frischwasser mehr in die Anmischung zugeführt werden muss.

Um noch weitere organische Substanz für den anaeroben Abbau verfügbar zu machen und damit die Biogasausbeute weiter zu erhöhen und/oder die anfallende Menge der feststoffhaltigen Phase zu reduzieren und/oder die Ausbeute an zurückgewonnenem Prozesswasser zu erhöhen, kann der Ablauf der Fermentation und/oder der gewonnene Feststoff der Phasentrennung einer biologischen Behandlung 10 zugeführt werden. Durch Zugabe von Enzymen und/oder Pilzen kann ein biologischer Aufschluss erreicht werden, wodurch besonders Cellulose und Hemicellulose für anaerobe Abbauprozesse leichter zugänglich werden und bei einer Rückführung des derart behandelten Stroms in die anaerobe Fermentation 9 zu Biogas umgesetzt werden können. Beim Einsatz nur von Enzymen sollte der TS-Gehalt des befeuchteten Substrats so gewählt werden, dass freies Wasser anwesend ist. In diesem Fall ist ein aerobes Milieu nicht immer zwingend erforderlich. Die Dosierung von einer oder mehrerer Enzymlösungen kann mit Dosierpumpen substratspezifisch erfolgen. Für ein optimales Wirken der Enzyme ist ein kontinuierliches oder diskontinuierliches Mischen des Substrates zum Beispiel in einem Rührbehälter von Vorteil. Die Wirkdauer der Enzyme beträgt zwischen 1-24h, vorzugsweise 2-6h, bei einer Temperatur zwischen 5-80°C, bevorzugt 20-50°C und einem pH-Wert zwischen 2-10, bevorzugt 5-8. Erfolgt die biologische Behandlung mit Pilzen, geschieht dies unter Einstellung, in der Regel unter Absenkung des pH-Wertes auf ca. 3,5 bis 8, besonders auf 4 bis 6 zum Beispiel durch Dosierung einer Säure. Die Dosierung der Pilze erfolgt zum Beispiel geregelt in Form einer pumpfähigen Zellsuspension. Als Reaktionsraum kommt zum Beispiel ein belüfteter Aerobreaktor in Betracht. Zusätzlich kann die biologische Behandlung 10 bei direkter Verwendung des Ablaufs der Fermentation 9 auch als aerobe Submerskultur ausgeführt werden. Dafür kann der Ablauf in einen belüfteten die ligninabbauenden Pilze enthaltenden kontinuierlich oder diskontinuierlich arbeitenden Aerobreaktor geleitet werden. Alternativ kann eine Einlagerung des befeuchteten und mit einer Pilzsuspension beaufschlagten Substrats in mehreren belüfteten Schlauchreaktoren erfolgen, welche einen quasikontinuierlichen Betrieb des Gesamtprozesses ermöglichen. Die Temperatur sollte dabei zwischen ca. 10°C und 40°C, bevorzugt im Bereich 15 bis 35°C liegen. Als Verweilzeit für eine werden ca. 1-4 Wochen, besonders ca. 1-2 Wochen bevorzugt.

Je nach Zielstellung wird der aus der biologischen Behandlung 10 austretende Strom in die Fermentation 9 zurückgeführt und/oder der Phasentrennung 11 zugeführt.

### Ausführungsbeispiel 2

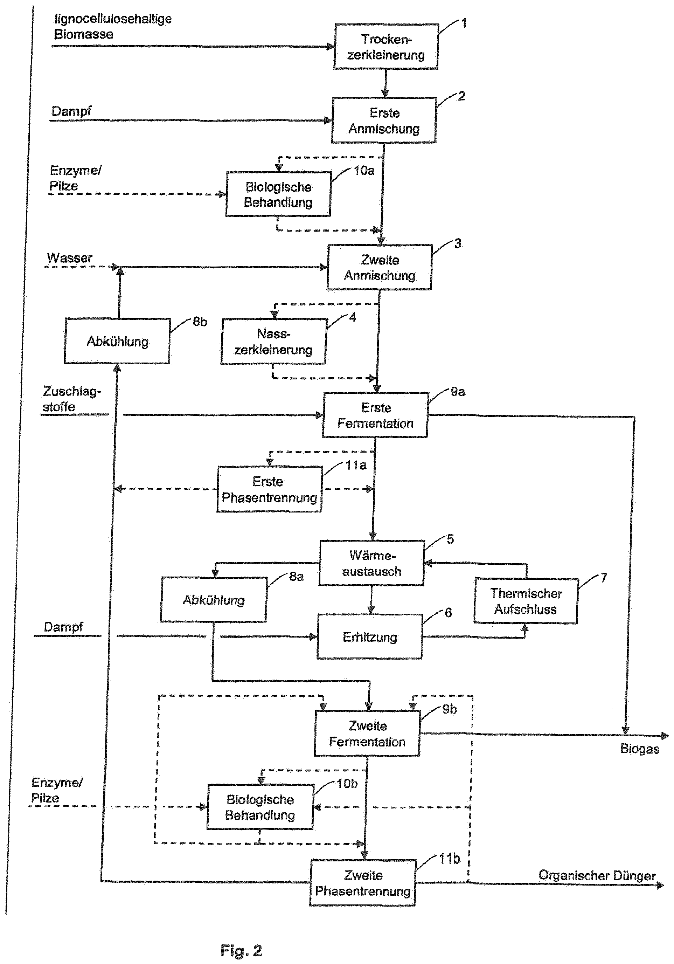

Fig. 2 zeigt ein schematisches Beispiel eines mehrstufigen Verfahrens zur Herstellung von Biogas aus lignocellulosehaltiger Biomasse wie zum Beispiel Stroh oder Holz gemäß der Erfindung.

Die lose oder gepresste lignocellulosehaltige Biomasse wird gegebenenfalls nach einer Reinigung von Steinen und sonstigen Fremdkörpern zunächst der Trockenzerkleinerung 1 zugeführt. In einem Schredder, einem Häcksler oder einer Hammermühle wird die lignocellulosehaltige Biomasse auf eine Substratpartikelgröße kleiner 200mm, bevorzugt kleiner 20mm, bei Stroh besonders bevorzugt auf eine Substratpartikelgröße zwischen 1mm und 10mm zerkleinert. Alternativ oder zusätzlich kann eine Bearbeitung in einem Nasszerkleinerer stattfinden.

Anschließend wird das zerkleinerte Substrat zum Beispiel pneumatisch in die erste Anmischung 2 gefördert. Die erste Anmischung 2 ist als Dämpfer, Mischer, Rührer oder Umwälzer ausgeführt. In diesem Apparat wird das zerkleinerte Substrat mit Heißwasser und/oder Dampf beaufschlagt, um es auf 60-100°C, bevorzugt auf 70-85°C, für eine Dauer von 0,5min bis 30min, bevorzugt 5min bis 15min zu erwärmen. Durch die erhöhten Temperaturen wird das Entgasen des Substrats erwirkt und die Aufnahmefähigkeit von Wasser verbessert, indem insbesondere eine Auflösung der Wachsschicht einsetzt. Durch die Wasseraufnahme des Substrats steigt die Dichte der Partikel, wodurch eine Schwimmschichtbildung in der Fermentation 9 reduziert oder sogar vollständig verhindert wird. In der ersten Anmischung 2 wird ein befeuchtetes Substrat mit einem TS-Gehalt zwischen 20% und 60%, bevorzugt ca. 30% generiert.

In einer Ausführungsform besteht die Möglichkeit einer biologischen Behandlung 10a des befeuchtetes Substrats, welches durch die hohen Temperaturen in der ersten Anmischung 2 pasteurisiert wurde. Durch Zugabe von Enzymen und/oder Pilzen kann ein biologischer Aufschluss erreicht werden, wodurch besonders Cellulose und Hemicellulose für die spätere anaerobe Fermentation leichter zugänglich werden. Beim Einsatz nur von Enzymen sollte der TS-Gehalt des befeuchteten Substrats so gewählt werden, dass freies Wasser anwesend ist. In diesem Fall ist ein aerobes Milieu nicht immer zwingend erforderlich. Die Dosierung von einer oder mehrerer Enzymlösungen kann mit Dosierpumpen substratspezifisch erfolgen. Für ein optimales Wirken der Enzyme ist ein kontinuierliches oder diskontinuierliches Mischen des Substrates zum Beispiel in einem Rührbehälter von Vorteil. Die Wirkdauer der Enzyme beträgt zwischen 1-24h, vorzugsweise 2-6h, bei einer Temperatur zwischen 5-80°C, bevorzugt 20-50°C und einem pH-Wert zwischen 2-10, bevorzugt 5-8. Erfolgt die biologische Behandlung mit Pilzen, geschieht dies unter Einstellung, in der Regel unter Absenkung des pH-Wertes auf ca. 3,5 bis 8, besonders auf 4 bis 6 zum Beispiel durch Dosierung einer Säure. Die Dosierung der Pilze erfolgt zum Beispiel geregelt in Form einer pumpfähigen Zellsuspension. Als Reaktionsraum kommt zum Beispiel ein belüfteter Aerobreaktor in Betracht. Alternativ kann die Einlagerung des befeuchteten und mit einer Pilzsuspension beaufschlagten Substrats in mehreren belüfteten Schlauchreaktoren erfolgen, welche einen quasikontinuierlichen Betrieb des Gesamtprozesses ermöglichen. Die Temperatur sollte dabei zwischen ca. 10°C und 40°C, bevorzugt im Bereich 15 bis 35°C liegen. Als Verweilzeit für eine werden ca. 1-4 Wochen, besonders ca. 1-2 Wochen bevorzugt.

Das durch die erste Anmischung 2 befeuchtete und gegebenenfalls auch durch die biologische Behandlung 10a aufgeschlossene Substrat wird in der zweiten Anmischung 3 zusammen mit Wasser zu einer Suspension mit einem TS-Gehalt zwischen 5% und 30%, bevorzugt zwischen 10% und 18% angemischt. Für das Anmischen werden bevorzugt Prozesswässer aus der Phasentrennung 11 verwendet, wodurch der Frischwasserbedarf fast vollständig oder sogar vollständig gedeckt werden kann. Zudem werden mit dem zurückgeführten Prozesswasser Nährstoffe, Spurenelemente und Mikroorganismen rezirkuliert.

Durch einen Kühler 8b zwischen der Phasentrennung 11b und der zweiten Anmischung 3 können die rückgeführten Prozesswässer so temperiert werden, dass die Temperatur der ersten Fermentation 9a in einem für den meso- oder thermophilen anaeroben Abbau geeigneten Temperaturbereich gehalten wird. In anderen Ausführungsformen kann der Kühler auch an anderer Stelle im Prozess installiert werden, um zum Beispiel den gesamten Flüssigkeitsstrom oder die Suspension nach dem zweiten Mischer zu kühlen.

In einigen Ausführungsformen kann es zudem sinnvoll sein, das in der Suspension eingemischte Substrat weiter zu zerkleinern, insbesondere wenn die mittlere Substratpartikelgröße größer als 2 mm ist. Dies kann mit einer Nasszerkleinerung 4 beispielsweise durch eine Nassmühle erreicht werden.

Die erzeugte Suspension wird gegebenenfalls zusammen mit Zuschlagstoffen einer ersten anaeroben Fermentation 9a zugeführt. Zuschlagstoffe können Stickstoffquellen beispielsweise in Form von Ammoniumhydrogencarbonat und/oder Makro- und Mikronährstoffen wie zum Beispiel Natrium- und/oder Phosphor- und/oder Zinkverbindungen und/oder kommerzielle Spurenelementlösungen sein. Die erste Fermentation 9a kann aus einem oder mehreren parallel betriebenen Fermentern bestehen, welche vorzugsweise mit einem Rührwerk ausgestattet sind und günstigerweise durch eine getaktete Substratzufuhr quasikontinuierlich betrieben werden. Die Verweilzeit der mesophilen oder thermophilen anaeroben Fermentation 9a beträgt vorzugsweise 10 bis 25 Tage. Das kontinuierlich erzeugte Biogas wird im oberen Teil des Fermenters bzw. der Fermenter abgezogen und über eine Sammelleitung zur weiteren Aufbereitung geführt.

Nachdem die lignocellulosehaltige Biomasse in der ersten Fermentation 9a zumindest teilweise zu Biogas vergoren wurde, wird der pumpfähige faserhaltige Ablauf einem kontinuierlich arbeitenden thermischen Aufschluss 7 zugeführt, um schwerer abbaubare Bestandteile für einen weiteren effizienten anaeroben Abbau zugänglich zu machen. Die Förderung in den unter Druck arbeitenden thermischen Aufschluss 7 kann zum Beispiel mit einer Exzenterschneckenpumpe erfolgen. Auch der Wärmetauscher 5 und die Erhitzung 6 sowie die Abkühlung 8a werden unter einem solchen Druck betrieben, dass aus dem wässrigen Medium kein Wasserdampf entsteht. Der thermische Aufschluss 7 erfolgt in wässriger Phase bei einer Temperatur von 130°C bis 200°C, bevorzugt bei einer Temperatur von 150°C bis 170°C. Um den energetischen Aufwand des thermischen Aufschlusses zu minimieren, erfolgt zunächst eine indirekte Aufwärmung des faserhaltigen Ablaufs in einem bevorzugt im Gegenstrom arbeitenden Wärmetauschers 5. Dabei wird Wärme vom bereits thermisch behandelten heißen Ablauf auf den noch zu behandelnden Ablauf übertragen. Der Wärmetauscher 5 überträgt dabei so viel Wärme, dass die Temperatur des erwärmten Ablaufs maximal 20K, bevorzugt maximal 10K unter der gewünschten Aufschlusstemperatur liegt. Im Anschluss erfolgt die weitere Erwärmung bis auf die Aufschlusstemperatur in der Erhitzung 6 mit Dampf. Dies kann indirekt zum Beispiel in einem Plattenwärmeübertrager oder durch Eindüsung von Direktdampf erfolgen. Schließlich strömt der heiße Ablauf in den Aufschlussreaktor (thermischer Aufschluss 7). In diesem isolierten Druckbehälter wird eine Verweilzeit von 5min bis 60min, vorzugsweise 10min bis 30min bei der entsprechenden Aufschlusstemperatur realisiert. Zur Verbesserung des Strömungsverhaltens kann der Aufschlussreaktor mit einem Rührwerk bzw. einer anderen Misch- oder Fördereinrichtung ausgestattet sein.

Es besteht in einer energetisch besonders vorteilhaften Form der Prozessführung die Möglichkeit den TS-Gehalt des zum thermischen Aufschluss 6 gelangenden Ablaufs zu erhöhen. Durch den geringeren Wassergehalt im zu behandelnden faserhaltigen Strom wird die benötigte Wärmemenge für den Aufschluss reduziert. Die Anreicherung der TS wird realisiert indem zumindest ein Teil des Ablaufs zunächst zur Phasentrennung 11a geführt und dort in eine flüssige und faserhaltige Phase getrennt wird. Diese Fest-Flüssig-Trennung kann zum Beispiel durch einen Dekanter realisiert werden. Die faserhaltige Phase wird gegebenenfalls nach Vermischung mit dem unbehandelten Teil des Ablaufs dem thermischen Aufschluss 6 zugeführt. Der bevorzugte TS-Gehalt des zum thermischen Aufschluss geförderten Ablaufs beträgt 7% bis 15%. Eine zurückgewonnene Flüssigphase aus der Phasentrennung 11a wird gegebenenfalls nach einer Zwischenspeicherung zur zweiten Anmischung 3 rezirkuliert.

Der heiße aufgeschlossene Ablauf passiert erneut den Wärmeaustausch 5, um die Wärme an den noch zu behandelnden Ablauf abzugeben. Danach wird der aufgeschlossene Ablauf in der Abkühlung 8a, welche zum Beispiel mit Kühlwasser arbeitet, so temperiert, dass die Temperatur der zweiten Fermentation 9b in einem für den meso- oder thermophilen anaeroben Abbau geeigneten Temperaturbereich gehalten wird.

Der aufgeschlossene faserhaltige Ablauf wird einer weiteren anaeroben Fermentation 9b zugeführt. Durch die weitere Vergärung der nun für die Biogaserzeugung besser verfügbaren Cellulose und Hemicellulose wird die Gesamtausbeute an Biogas deutlich gesteigert. Die Verweilzeit der mesophilen oder thermophilen anaeroben Fermentation 9b beträgt vorzugsweise 5 bis 15 Tage. Auch die zweite Fermentation 9b kann aus einem oder mehreren parallel betriebenen Fermentern bestehen, welche vorzugsweise mit einem Rührwerk ausgestattet sind und günstigerweise durch eine getaktete Substratzufuhr quasikontinuierlich betrieben werden. Das kontinuierlich erzeugte Biogas wird im oberen Teil des Fermenters bzw. der Fermenter abgezogen und über eine Sammelleitung zusammen mit dem Gas der ersten Fermentation 9a zur weiteren Aufbereitung geführt.

Der Ablauf aus der zweiten Fermentation 9b enthält nur noch sehr schwer abbaubare Faserbestandteile, welche zu großen Teilen aus Lignin bestehen. Durch den Mineraliengehalt und die noch enthaltenen organischen Bestandteile kann der Ablauf sehr gut als organischer Dünger eingesetzt werden. Vorzugsweise wird dieser zweite Ablauf jedoch zunächst einer zweiten Phasentrennung 11b zugeführt und in eine flüssige und eine feststoffhaltige Phase getrennt. Auch diese Phasentrennung kann zum Beispiel mit einem Dekanter realisiert werden. In diesem Fall wird eine stichfeste, gut lagerfähige feste Phase mit kaum vorhandenem oder gar keinem freien Wasser erzeugt. Die feststoffhaltige Phase kann zur weiteren Ausbeutesteigerung anteilig in die zweite Fermentation zurückgeführt werden. Dadurch wird eine höhere Feststoffverweilzeit erreicht und die Biogasausbeute erhöht. Der überschüssige Anteil der feststoffhaltigen Phase kann gegebenenfalls nach einer Zwischenlagerung als organischer Dünger in der Landwirtschaft verwendet werden. Dadurch kann die Humusbilanz und Mineralstoffzufuhr auf die Agrarflächen wesentlich verbessert und eine nachhaltige Bewirtschaftung sichergestellt werden.

Die in der zweiten Phasentrennung 11b erzeugte flüssige Phase wird als Prozesswasser gegebenenfalls nach einer Zwischenlagerung und einer Abkühlung (8b) in die zweite Anmischung 3 zurückgeführt. Durch den effektiven Abbau des Substrats zu Biogas wird gleichzeitig so viel TS abgebaut, dass in der Phasentrennung 11 viel Prozesswasser generiert wird, wodurch der Wasserbedarf des Gesamtprozesses der Biogaserzeugung aus lignocellulosehaltiger Biomasse besonders gering ist. Die rezirkulierte Menge Prozesswasser kann so hoch sein, dass gar kein Frischwasser mehr in die Anmischung zugeführt werden muss.

Um noch weitere organische Substanz für den anaeroben Abbau verfügbar zu machen und damit die Biogasausbeute weiter zu erhöhen und/oder die anfallende Menge der feststoffhaltigen Phase zu reduzieren und/oder die Ausbeute an zurückgewonnenem Prozesswasser zu erhöhen, kann der Ablauf der zweiten Fermentation und/oder der gewonnene Feststoff der zweiten Phasentrennung einer biologischen Behandlung 10b zugeführt werden. Diese kann vorzugsweise mit ligninabbauenden Pilzen analog der biologischen Behandlung 10a realisiert werden. Zusätzlich kann die biologische Behandlung 10b bei direkter Verwendung des Ablaufs der zweiten Fermentation 9b auch als aerobe Submerskultur ausgeführt werden. Dafür kann der Ablauf in einen belüfteten die ligninabbauenden Pilze enthaltenden kontinuierlich oder diskontinuierlich arbeitenden Aerobreaktor geleitet werden. Je nach Zielstellung wird der aus der biologischen Behandlung 10b austretende Strom in die zweite Fermentation 9b zurückgeführt und/oder der zweiten Phasentrennung 11b zugeführt.

## Patentansprüche

1. Verfahren zur Herstellung von Biogas aus lignocellulosehaltiger Biomasse, vorzugsweise aus Stroh, welches folgende Schritte umfasst:
a) Anmischen der lignocellulosehaltigen Biomasse mit einer durchschnittlichen Partikelgröße kleiner als 200mm, vorzugsweise kleiner als 20mm, mit wässrigen Lösungen bei einer Temperatur von 60-100°C, insbesondere von 70-85°C, für eine Zeitdauer von 5-30min insbesondere mit Prozesswasser und/oder unter Zuhilfenahme von Dampf;
a1) anaerobe Fermentation der angemischten lignocellulosehaltigen Biomasse mithilfe von Mikroorganismen und Gewinnung des entstehenden Biogases
a2) Durchführung eines im Gegenstromprinzip arbeitenden Wärmeaustauschs zwischen der in Schritt a1) erhaltenen einer ersten anaeroben Fermentation unterzogenen lignocellulosehaltigen Biomasse und der in Schritt b) erhaltenen thermisch behandelten lignocellulosehaltigen Biomasse;
b) weitere Erhitzung der aus a2) im Gegenstrom erwärmten lignocellulosehaltigen Biomasse auf 130-200°C, vorzugsweise 150-180°C, und Verweilen der so erhitzten lignocellulosehaltigen Biomasse für eine Dauer von 5-120min, bevorzugt 10-30min, zweckmäßig in einem kontinuierlich durchströmten Reaktorraum zum thermischen Aufschluss;
c) anaerobe Fermentation der thermisch behandelten lignocellulosehaltigen Biomasse mithilfe von Mikroorganismen und Gewinnung des entstehenden Biogases;
d) wobei der Ablauf nach der Fermentation c), zumindest anteilig einer Phasentrennung unterzogen wird und
e) eine flüssige Phase aus d) in Form von Prozesswasser gewonnen wird und diese zum Anmischen von lignocellulosehaltiger Biomasse rückgeführt wird derart, dass die Zufuhr an Frischwasser im Biogaserzeugungsprozess und/oder zum Anmischen zumindest wesentlich reduziert wird oder vollständig entfällt sowie
f) eine feststoffreiche Phase aus d) zum Beispiel als organischer Dünger anfällt.

2. Verfahren gemäß Anspruch 1,
**dadurch gekennzeichnet, dass** vor Durchführung des Schrittes a2) eine vorherige Einstellung des Trockensubstanzgehaltes auf 7-20 Gew.-% erfolgt.

3. Verfahren gemäß Anspruch 1,
**dadurch gekennzeichnet, dass** durch den im Gegenstrom arbeitenden Wärmeaustausch des Schrittes a2) eine Vorerwärmung der in Schritt a1) erhaltenen lignocellulosehaltigen Biomasse auf eine Temperatur von 110-180°C, insbesondere 130-160°C, erfolgt.

4. Verfahren nach mindestens einem der vorangegangenen Ansprüche,
wobei die anaerobe Fermentation zusammen mit der Einstellung des Natriumgehaltes auf 100mg/l bis 5000mg/l beispielsweise durch Dosierung von Natriumverbindungen erfolgt.

5. Verfahren nach mindestens einem der vorangegangenen Ansprüche,
wobei die anaerobe Fermentation zusammen mit der Einstellung des Ammoniumgehaltes auf 200mg/l bis 2000mg/l beispielsweise durch Dosierung von Ammoniumverbindungen erfolgt.

6. Verfahren nach mindestens einem der vorangegangenen Ansprüche,
wobei der Ablauf nach Beendigung der gesamten Fermentation zumindest anteilig einer Phasentrennung unterzogen wird und eine feststoffreiche Phase als organischer Dünger derart anfällt, dass die Verwendung des organischen Düngers eine nachhaltige Bewirtschaftung durch Gewährleistung der Humusreproduktion und/oder Schließung des Nährstoffkreislaufes von agrarischen Anbauflächen erlaubt.

7. Verfahren nach mindestens einem der vorangegangenen Ansprüche,
wobei Teile des lignocellulosehaltigen Ablaufs nach Beendigung der gesamten Fermentation und/oder Teile der feststoffreichen Phase einer zumindest anteiligen Phasentrennung des lignocellulosehaltigen Ablaufs vorzugsweise nach einer Pasteurisierung einer biologischen Behandlung mit ligninabbauenden Pilzen unterzogen werden, um nach erneuter Zuführung in eine anaerobe Fermentation zur weiteren Biogasbildung beizutragen und/oder um nach anschließender Phasentrennung zur Gewinnung einer flüssigen Phase in Form von Prozesswasser zur Rückführung in den Biogaserzeugungsprozess, insbesondere zum Anmischen von lignocellulosehaltiger Biomasse, beizutragen.

8. Verfahren nach mindestens einem der vorangegangenen Ansprüche,
wobei die lignocellulosehaltige Biomasse während und/oder nach einem Anmischen mit Wasser, insbesondere in Form von Prozesswasser und/oder unter Zuhilfenahme von Dampf unter der Anwesenheit von freiem Wasser einer biologischen Behandlung mit Enzymen derart unterzogen wird, dass Cellulose- und/oder Hemicellulosebestandteile in einer nachfolgenden anaeroben Fermentation wesentlich besser verfügbar sind.

9. Verfahren nach mindestens einem der vorangegangenen Ansprüche,
wobei die lignocellulosehaltige Biomasse nach einem Anmischen mit Wasser, insbesondere in Form von Prozesswasser und/oder unter Zuhilfenahme von Dampf einer biologischen Behandlung mit Pilzen derart unterzogen wird, dass Cellulose- und/oder Hemicellulosebestandteile in einer nachfolgenden anaeroben Fermentation wesentlich besser verfügbar sind.

10. Verfahren nach einem der vorhergehenden Ansprüche, wobei der lignocellulosehaltigen Biomasse weitere organische Stoffe beigemengt werden, insbesondere Dung oder Gülle, wobei die Zugabe der weiteren organischen Stoffe vor oder während des Anmischens der lignocellulosehaltigen Biomasse in Schritt a) und/oder vor oder während der anaeroben Fermentation gemäß einem der Verfahrensschritte a1) und/oder c) erfolgt.

11. Verfahren nach einem der vorherigen Ansprüche,
**dadurch gekennzeichnet, dass** das Anmischen der lignocellulosehaltigen Biomasse bei einer Temperatur von 60-100°C, insbesondere 70-85°C für eine Zeitdauer von 5 bis 15 Minuten erfolgt.

## Claims

1. A method for producing biogas from lignocellulose-containing biomass, preferably from straw, which comprises the following steps:
a) mixing the lignocellulose-containing biomass with an average particle size smaller than 200 mm, preferably smaller than 20 mm, with aqueous solutions at a temperature of 60-100°C, especially 70-85°C, for a duration of 5-30 mins, especially with process water and/or with the aid of steam;
a1) anaerobically fermenting the mixed, lignocellulose-containing biomass by means of microorganisms, and recovering the biogas formed;
a2) performing a heat exchange operating on the countercurrent principle between the lignocellulose-containing biomass subjected to a first anaerobic fermentation obtained in step a1) and the heat-treated lignocellulose-containing biomass obtained in step b);
b) further heating the lignocellulose-containing biomass heated in countercurrent from a2) to 130-200°C, preferably 150-180°C, and leaving the thus heated lignocellulose-containing biomass for a duration of 5-120 mins, preferably 10-30 mins, advantageously in a continuous flow reactor space for thermal digestion;
c) anaerobically fermenting the heat-treated lignocellulose-containing biomass by means of microorganisms, and recovering the biogas formed;
d) wherein the outflow after the fermentation c) is subjected at least in part to a phase separation; and
e) a liquid phase in the form of process water is obtained from d), and this is recycled to the mixing of lignocellulose-containing biomass, in such a manner that the supply of fresh water in the biogas generation process and/or for mixing is at least considerably reduced or becomes completely unnecessary; and
f) a solids-rich phase is formed from d), for example as organic fertilizer.

2. A method according to claim 1,
**characterized in that**
before step a2) is performed, there is a prior adjustment of the dry substance content to 7-20 wt.%.

3. A method according to claim 1,
**characterized in that**
through the heat exchange operating in countercurrent as per step a2), the lignocellulose-containing biomass obtained in step a1) is preheated to a temperature of 110-180°C, especially 130-160°C.

4. A method according to at least one of the preceding claims, wherein the anaerobic fermentation is effected together with the adjustment of the sodium content to 100 mg/l up to 5000 mg/l, for example by introduction of sodium compounds.

5. A method according to at least one of the preceding claims, wherein the anaerobic fermentation is effected together with the adjustment of the ammonium content to 200 mg/l up to 2000 mg/l, for example by introduction of ammonium compounds.

6. A method according to at least one of the preceding claims, wherein the outflow after ending of the whole fermentation is at least in part subjected to a phase separation and a solids-rich phase is formed as organic fertilizer such that the use of the organic fertilizer allows sustainable management by ensuring humus reproduction and/or closure of the nutrient cycle of agricultural farmland.

7. A method according to at least one of the preceding claims, wherein parts of the lignocellulose-containing outflow after ending of the whole fermentation and/or parts of the solids-rich phase from an at least partial phase separation of the lignocellulose-containing outflow preferably after a pasteurization are subjected to a biological treatment with lignin-degrading fungi, in order, after renewed introduction into an anaerobic fermentation, to contribute to further biogas formation and/or in order, after subsequent phase separation, to contribute to the recovery of a liquid phase in the form of process water for recycling into the biogas generation process, especially for the mixing of lignocellulose-containing biomass.

8. A method according to at least one of the preceding claims, wherein the lignocellulose-containing biomass during and/or after mixing with water, especially in the form of process water and/or with the aid of steam, is subjected to a biological treatment with enzymes in the presence of free water in such a manner that cellulose and/or hemicellulose components are considerably better available in a subsequent anaerobic fermentation.

9. A method according to at least one of the preceding claims, wherein the lignocellulose-containing biomass after mixing with water, especially in the form of process water and/or with the aid of steam, is subjected to a biological treatment with fungi in such a manner that cellulose components and/or hemicellulose components are considerably better available in a subsequent anaerobic fermentation.

10. A method according to one of the preceding claims, wherein further organic substances, especially dung or liquid manure, are mixed into the lignocellulose-containing biomass, the addition of the further organic substances being effected before or during the mixing of the lignocellulose-containing biomass in step a) and/or before or during the anaerobic fermentation according to one of the method steps a1) and/or c).

11. A method according to one of the preceding claims, **characterized in that** the mixing of the lignocellulose-containing biomass is effected at a temperature of 60-100°C, especially 70-85°C for a duration of from 5 to 15 minutes.

## Revendications

1. Procédé de production de biogaz à partir de biomasse lignocellulosique, de préférence de paille, comprenant les étapes suivantes consistant à :
a) mélanger la biomasse lignocellulosique ayant une taille moyenne de particules inférieure à 200 mm, de préférence inférieure à 20 mm, avec des solutions aqueuses à une température de 60 à 100°C, en particulier de 70 à 85°C, pendant une durée de 5 à 30 min, en particulier avec de l'eau de traitement et/ou à l'aide de vapeur ;
a1) procéder à la fermentation anaérobie de la biomasse lignocellulosique mélangée à l'aide de micro-organismes et extraire le biogaz généré,
a2) réaliser un échange de chaleur fonctionnant selon le principe du contre-courant entre la biomasse lignocellulosique, obtenue à l'étape a1), soumise à une première fermentation anaérobie, et la biomasse lignoscellulosique obtenue à l'étape b), traitée thermiquement ;
b) chauffer une nouvelle fois à 130-200°C, de préférence à 150-180°C, la biomasse lignocellulosique chauffée à contre-courant provenant du a2), et laisser utilement la biomasse lignocellulosique ainsi chauffée pendant une durée de 5 à 120 min, de préférence 10 à 30 min, dans une chambre de réacteur traversée en continu jusqu'à la digestion thermique ;
c) effectuer la fermentation anaérobie de la biomasse lignocellulosique traitée thermiquement à l'aide de micro-organismes et extraire le biogaz généré ;
d) l'écoulement après la fermentation c) étant au moins en partie soumis à une séparation de phase, et
e) une phase liquide provenant de d) étant extraite sous forme d'eau de traitement et puis renvoyée au mélange de biomasse lignocellulosique de telle sorte que l'apport en eau fraîche dans le procédé de production de biogaz et/ou pour le mélange soit au moins sensiblement réduit voire totalement supprimé et
f) une phase riche en matières solides provenant de d) étant obtenue par exemple en tant qu'engrais organique.

2. Procédé selon la revendication 1, **caractérisé en ce que**, avant la réalisation de l'étape a2), un ajustement préalable de la teneur en matières sèches de 7 à 20 % en poids s'effectue.

3. Procédé selon la revendication 1, **caractérisé en ce qu'**un préchauffage de la biomasse lignocellulosique obtenue à l'étape a1) s'effectue à une température de 110 à 180°C, en particulier de 130 à 160°C par l'échange de chaleur de l'étape a2) fonctionnant à contre-courant.

4. Procédé selon au moins l'une des revendications précédentes, dans lequel la fermentation anaérobie s'effectue conjointement avec l'ajustement de la teneur en sodium à 100 mg/l jusqu'à 5000 mg/l, par exemple par ajout de composés de sodium.

5. Procédé selon au moins l'une des revendications précédentes, dans lequel la fermentation anaérobie s'effectue conjointement avec l'ajustement de la teneur en ammonium à 200 mg/l jusqu'à 2000 mg/l, par exemple par ajout de composés d'ammonium.

6. Procédé selon au moins l'une des revendications précédentes, dans lequel l'écoulement après achèvement de la fermentation totale est au moins en partie soumis à une séparation de phase et une phase riche en matières solides est obtenue en tant qu'engrais organique de sorte que l'utilisation de l'engrais organique autorise une exploitation ultérieure en garantissant la production d'humus et/ou en fermant le circuit des nutriments des surfaces de culture agraires.

7. Procédé selon au moins l'une des revendications précédentes, dans lequel des parties de l'écoulement lignocellulosique après achèvement de la fermentation totale et/ou des parties de la phase riche en matières solides sont soumises à une séparation de phase au moins proportionnelle de l'écoulement lignocellulosique, de préférence après une pasteurisation d'un traitement biologique avec des champignons dégradant la lignine pour contribuer à une formation de biogaz ultérieure après une nouvelle amenée à une fermentation anaérobie et/ou pour contribuer après une séparation de phase consécutive à l'extraction d'une phase liquide sous la forme d'eau de traitement pour le renvoi dans le procédé de production de biogaz, en particulier pour le mélange de la biomasse lignocellulosique.

8. Procédé selon au moins l'une des revendications précédentes, dans lequel la biomasse lignocellulosique est soumise pendant et/ou après un mélange avec l'eau, en particulier sous la forme d'eau de traitement et/ou à l'aide de vapeur en présence d'eau libre, à un traitement biologique avec des enzymes de telle sorte que les composants de cellulose et/ou d'hémicellulose soient sensiblement mieux disponibles au cours d'une fermentation anaérobie suivante.

9. Procédé selon au moins l'une des revendications précédentes, dans lequel la biomasse lignocellulosique est soumise après un mélange avec l'eau, en particulier sous la forme d'eau de traitement et/ou à l'aide de vapeur, à un traitement biologique avec des champignons de telle sorte que les composants de cellulose et/ou d'hémicellulose soient sensiblement mieux disponibles au cours d'une fermentation anaérobie suivante.

10. Procédé selon l'une des revendications précédentes, dans lequel la biomasse lignocellulosique est additionnée d'autres substances organiques, en particulier d'engrais ou de purin, où l'addition d'autres substances organiques s'effectue avant ou pendant le mélange de la biomasse lignocellulosique à l'étape a) et/ou avant ou pendant la fermentation anaérobie selon l'une des étapes de procédé a1) et/ou c).

11. Procédé selon l'une des revendications précédentes, **caractérisé en ce que** le mélange de la biomasse lignocellulosique s'effectue à une température de 60 à 100°C, en particulier de 70 à 85°C, pendant une durée de 5 à 15 minutes.
